# EUROPEAN PATENT APPLICATION

(11) **EP 3 567 056 A1**
(43) Date of publication of application: **13.11.2019**
(21) Application number: 19175629.5
(22) Date of filing: 04.09.2015
(51) Int. Cl.: C07K 16/32, A61P 35/00, C12P 21/00

(54) **GALACTOENGINEERED IMMUNOGLOBULIN 1 ANTIBODIES**

(30) Priority: 10.09.2014 EP 14184201; 23.12.2014 US 201462095912 P
(62) Divisional of application: 15759788.1
(71) Applicant: F. Hoffmann-La Roche AG, 4070 Basel (CH)
(72) Inventor: MALIK, Sebastian, 82377 Penzberg (DE); REUSCH, Dietmar, 82377 Penzberg (DE); SCHNUERIGER, Alfred, deceased (DE); TEJADA, Max L., South San Francisco, California 94080 (US); THOMANN, Marco, 82377 Penzberg (DE)
(74) Representative: Mertes, Maria Margot Martha

(57) **Abstract**

The present invention relates to galactoengineered recombinant antibodies of IgG1 isotype, methods for the production of said antibodies and uses thereof.

SEQUENCE LISTING

<110>  F. Hoffmann-La Roche AG

<120>  GALACTOENGINEERED IMMUNOGLOBULIN 1 ANTIBODIES

<130>  P32306-EP-2

<150>  EP 14184201.3
<151>  2014-09-10

<150>  US 62/095912
<151>  2014-12-23

<160>  10

<170>  PatentIn version 3.5

<210>  1
<211>  120
<212>  PRT
<213>  Artificial Sequence

<220>
<223>  Heavy chain variable domain of antibody trastuzumab

<400>  1


<210>  2
<211>  107
<212>  PRT

<213>  Artificial Sequence

<220>
<223>  Light chain variable domain of antibody trastuzumab

<400>  2

<210>  3
<211>  120
<212>  PRT
<213>  Artificial Sequence

<220>
<223>  Heavy chain variable domain of antibody rituximab

<400>  3

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser
        115                 120

<210>  4
<211>  107
<212>  PRT
<213>  Artificial Sequence

<220>
<223>  Light chain variable domain of antibody rituximab

<400>  4

Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Ile
            20                  25                  30

His Trp Phe Gln Gln Lys Pro Gly Ser Ser Pro Lys Pro Trp Ile Tyr
        35                  40                  45

Ala Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Thr Ser Asn Pro Pro Thr
                85                  90                  95

Phe Gly Gly Gly Thr Lys Leu Glu Ile Lys Arg
            100                 105

<210>  5
<211>  120
<212>  PRT
<213>  Artificial Sequence

<220>
<223>  Heavy chain variable domain of antibody pertuzumab

<400>  5

Glu Val Gln Leu Val Glu Ser Gly Gly Gly Leu Val Gln Pro Gly Gly

1                   5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

Thr Leu Val Thr Val Ser Ser Ala
        115                 120

<210>  6
<211>  106
<212>  PRT
<213>  Artificial Sequence

<220>
<223>  Light chain variable domain of antibody pertuzumab

<400>  6

Asp Ile Gln Met Thr Gln Ser Pro Ser Ser Leu Ser Ala Ser Val Gly
1                   5                   10                  15

Asp Arg Val Thr Ile Thr Cys Lys Ala Ser Gln Asp Val Ser Ile Gly
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile
            100                 105

<210>  7
<211>  120
<212>  PRT
<213>  Artificial Sequence

<220>
<223>  Heavy chain variable domain of antibody obinutuzumab

<400>  7

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala
        115                 120

<210>  8
<211>  108
<212>  PRT
<213>  Artificial Sequence

<220>
<223>  Light chain variable domain of antibody obinutuzumab

<400>  8

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser

20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys
            100                 105

<210>  9
<211>  306
<212>  PRT
<213>  Homo sapiens

<400>  9

Glu Ala Ser Phe Gln Val Trp Asn Lys Asp Ser Ser Ser Lys Asn Leu
1               5                   10                  15

Ile Pro Arg Leu Gln Lys Ile Trp Lys Asn Tyr Leu Ser Met Asn Lys
            20                  25                  30

Tyr Lys Val Ser Tyr Lys Gly Pro Gly Pro Gly Ile Lys Phe Ser Ala
        35                  40                  45

Glu Ala Leu Arg Cys His Leu Arg Asp His Val Asn Val Ser Met Val
    50                  55                  60

Glu Val Thr Asp Phe Pro Phe Asn Thr Ser Glu Trp Glu Gly Tyr Leu
65                  70                  75                  80

Pro Lys Glu Ser Ile Arg Thr Lys Ala Gly Pro Trp Gly Arg Cys Ala
                85                  90                  95

Val Val Ser Ser Ala Gly Ser Leu Lys Ser Ser Gln Leu Gly Arg Glu
            100                 105                 110

Ile Asp Asp His Asp Ala Val Leu Arg Phe Asn Gly Ala Pro Thr Ala
        115                 120                 125

Asn Phe Gln Gln Asp Val Gly Thr Lys Thr Thr Ile Arg Leu Met Asn

130                 135                 140

Ser Gln Leu Val Thr Thr Glu Lys Arg Phe Leu Lys Asp Ser Leu Tyr
145                 150                 155                 160

Asn Glu Gly Ile Leu Ile Val Trp Asp Pro Ser Val Tyr His Ser Asp
                165                 170                 175

Ile Pro Lys Trp Tyr Gln Asn Pro Asp Tyr Asn Phe Phe Asn Asn Tyr
            180                 185                 190

Lys Thr Tyr Arg Lys Leu His Pro Asn Gln Pro Phe Tyr Ile Leu Lys
        195                 200                 205

Pro Gln Met Pro Trp Glu Leu Trp Asp Ile Leu Gln Glu Ile Ser Pro
    210                 215                 220

Glu Glu Ile Gln Pro Asn Pro Pro Ser Ser Gly Met Leu Gly Ile Ile
225                 230                 235                 240

Ile Met Met Thr Leu Cys Asp Gln Val Asp Ile Tyr Glu Phe Leu Pro
                245                 250                 255

Ser Lys Arg Lys Thr Asp Val Cys Tyr Tyr Tyr Gln Lys Phe Phe Asp
            260                 265                 270

Ser Ala Cys Thr Met Gly Ala Tyr His Pro Leu Leu Tyr Glu Lys Asn
        275                 280                 285

Leu Val Lys His Leu Asn Gln Gly Thr Asp Glu Asp Ile Tyr Leu Leu
    290                 295                 300

Gly Lys
305

<210>  10
<211>  298
<212>  PRT
<213>  Homo sapiens

<400>  10

Leu Gln Lys Ile Trp Lys Asn Tyr Leu Ser Met Asn Lys Tyr Lys Val
1               5                   10                  15

Ser Tyr Lys Gly Pro Gly Pro Gly Ile Lys Phe Ser Ala Glu Ala Leu
            20                  25                  30

Arg Cys His Leu Arg Asp His Val Asn Val Ser Met Val Glu Val Thr

        35                  40                  45

Asp Phe Pro Phe Asn Thr Ser Glu Trp Glu Gly Tyr Leu Pro Lys Glu
    50                  55                  60

Ser Ile Arg Thr Lys Ala Gly Pro Trp Gly Arg Cys Ala Val Val Ser
65                  70                  75                  80

Ser Ala Gly Ser Leu Lys Ser Ser Gln Leu Gly Arg Glu Ile Asp Asp
                85                  90                  95

His Asp Ala Val Leu Arg Phe Asn Gly Ala Pro Thr Ala Asn Phe Gln
            100                 105                 110

Gln Asp Val Gly Thr Lys Thr Thr Ile Arg Leu Met Asn Ser Gln Leu
        115                 120                 125

Val Thr Thr Glu Lys Arg Phe Leu Lys Asp Ser Leu Tyr Asn Glu Gly
    130                 135                 140

Ile Leu Ile Val Trp Asp Pro Ser Val Tyr His Ser Asp Ile Pro Lys
145                 150                 155                 160

Trp Tyr Gln Asn Pro Asp Tyr Asn Phe Phe Asn Asn Tyr Lys Thr Tyr
                165                 170                 175

Arg Lys Leu His Pro Asn Gln Pro Phe Tyr Ile Leu Lys Pro Gln Met
            180                 185                 190

Pro Trp Glu Leu Trp Asp Ile Leu Gln Glu Ile Ser Pro Glu Glu Ile
        195                 200                 205

Gln Pro Asn Pro Pro Ser Ser Gly Met Leu Gly Ile Ile Ile Met Met
    210                 215                 220

Thr Leu Cys Asp Gln Val Asp Ile Tyr Glu Phe Leu Pro Ser Lys Arg
225                 230                 235                 240

Lys Thr Asp Val Cys Tyr Tyr Tyr Gln Lys Phe Phe Asp Ser Ala Cys
                245                 250                 255

Thr Met Gly Ala Tyr His Pro Leu Leu Tyr Glu Lys Asn Leu Val Lys
            260                 265                 270

His Leu Asn Gln Gly Thr Asp Glu Asp Ile Tyr Leu Leu Gly Lys Ala
        275                 280                 285

Thr Leu Pro Gly Phe Arg Thr Ile His Cys
    290                 295

## Description

### FIELD OF THE INVENTION

The present invention relates to galactoengineered recombinant antibodies of IgG1 isotype, methods for the production of said antibodies and uses thereof.

### BACKGROUND OF THE INVENTION

IgGs are the most abundant antibody isotypes, with IgG1 antibodies being the subclass exhibiting the most significant degree and array of effector functions. IgG1 antibodies are the most commonly used antibodies in immunotherapy, where ADCC and CDC are often deemed important. Within the structure of the antibody, the CH2 domain as well as the IgG hinge region plays a major role in Fc mediated antibody effector functions. Each CH2 domain comprises a conserved glycosylation site at an asparagine residue located at about position 297 (numbering according to EU index of Kabat), at which a glycan moiety is covalently bound (Wright, A. and Morrison, S.L., TIBTECH 15 (1997) 26-32). In the mature IgG molecule, the glycans are buried between the CH2 domains, influencing the tertiary structure of the IgG molecule. The N-linked glycans present in the Fc region of an antibody are known to be essential for the antibody to mediate effector functions such as ADCC (Lifely, M.R. et al. Glycobiology. 1995 Dec;5(8):813-22;, Jefferis R. et al. Immunol Rev. 1998 Jun;163:59-76.).

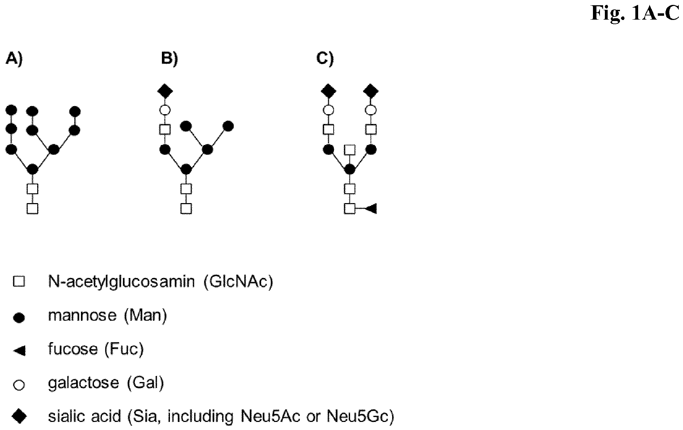

In general, three major types of N-linked glycans are known, including high-mannose structures (Fig. 1A), complex biantennary structures (Fig. 1C) and hybrid structures (Fig. 1B) with a branch of mannose residues and a complex branch. The glycans of the Fc region of antibodies predominantly are highly heterogeneous complex biantennary structures as indicated in Fig. 1C. While further non-conserved glycosylation sites may be present within the Fab region of an antibody, the influence of antibody glycosylation on its effector functions has been attributed to Fc glycosylation. It has been shown that the composition of the N-linked glycan affects the structure of the Fc region of the IgG molecule and thereby alters antibody effector functions such as Fc-receptor binding, ADCC activity and CDC activity (Presta, L. Curr Opin Struct Biol. 2003 Aug;13(4):519-25). Within IgG antibodies expressed in recombinant expression systems, e.g. by expression in prokaryotic or eukaryotic host cells, the N-linked glycan structure varies between individual antibody molecules. Therefore, antibodies produced in recombinant expression systems can be considered a "population of antibodies" (a term that is further used herein), with antibodies being identical in their amino acid sequence but exhibiting heterogeneity with respect to the N-linked glycan pattern of their Fc region.

The composition of the Fc glycans is known to vary between different host cell species used for expression of recombinant antibodies. Two commonly used host cell lines for the recombinant expression of antibodies are Chinese hamster ovary cells (CHO cells) and mouse myeloma cells (e.g. sp2/0, P3X63Ag8.653, NS0). CHO cells express recombinant antibodies, which are substantially devoid of terminal sialic acid residues, while a major fraction of the glycan patterns are fucosylated. In contrast, mouse myeloma cells give rise to antibody populations with up to 50 % (relative frequency) of sialic acid residues but with less of fucose residues.

It is known that some of the terminal residues of the glycan structure influence the IgG effector functions. The presence of a terminal fucose residue is known to contribute to reduced Fc-gammaRIIIa binding and to reduced ADCC. Hence, antibodies lacking terminal fucose residues ("afucosylated" antibodies) are associated with an increase of ADCC mediated by the antibody population. While the influence of afucosylation on improvement of ADCC mediation is been widely accepted within the art, the role of Fc galactosylation in ADCC mediation is controversially reported. Several studies indicate that galactosylation has no effect on ADCC (Boyd, P.N. et al. Mol Immunol. 1995 Dec;32(17-18):1311-8; Hodoniczky J. et al. Biotechnol Prog. 2005 Nov-Dec;21(6):1644-52; Raju T.S. Curr Opin Immunol. 2008 Aug;20(4):471-8); whereas other studies do report that galactosylation of IgG increases Fc-gammaRIIIa binding (Houde D. et al. Mol Cell Proteomics. 2010 Aug;9(8):1716-28; Kumpel B.M. et al. Hum Antibodies Hybridomas. 1995;6(3):82-8).

Currently, engineering of IgG molecules in order to improve ADCC mediated by the antibodies focuses on adjusting the fucosylation of IgG molecules. Afucosylation of recombinantly expressed IgG may be achieved by expressing antibodies in genetically engineered host cells, e.g. Lec13 CHO cells deficient in protein fucosylation or knockout cell lines, such as CHO cells with a knockout of the alpha-1,6-fucosyltransferase (*FUT8*) gene.

However, antibodies generated by current expression systems, e.g. CHO cells, exhibit a heterogeneous glycan pattern, leading to variations in the distribution of the distinct glycan species within different batches of generated antibodies.

Therefore, there is still a need for tailoring effector functions of recombinant IgG antibodies, especially for the provision of means for improving ADCC mediated by therapeutic antibodies.

### SUMMARY OF THE INVENTION

The present invention relates to a population of galactoengineered recombinant antibodies of IgG1 isotype, comprising a relative frequency of at least 80 % of Fc galactosylated antibodies.

The present invention also relates to a population of galactoengineered recombinant antibodies of IgG1 isotype, comprising a relative frequency of at least 70 % of Fc bi-galactosylated antibodies and a relative frequency of less than 20 % of Fc afucosylated antibodies.

One embodiment of the invention relates to a population of antibodies, wherein the antibodies do not comprise a glycosylation site in their Fab fragment.

One embodiment of the invention relates to a population of antibodies, wherein a mode of action of said antibody is the induction of ADCC.

One embodiment of the invention relates to a population of antibodies, wherein the antibody is selected from the group consisting of trastuzumab, rituximab, pertuzumab and obinutuzumab.

Another aspect of the invention is a method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) genetically engineering the host cell to improve protein galactosylation such that a population of said galactoengineered recombinant antibody is obtained, which comprises a relative frequency of at least 70 % of Fc bi-galactosylated antibodies.

Another aspect of the invention is a method for the production of a population of galactoengineered antibodies, comprising the steps of
a) recombinantly producing an antibody of IgG1 isotype in a host cell, which comprises nucleic acid molecules encoding the antibody, to obtain a population of said recombinant antibody,
b) isolating said population of the recombinant antibody,
c) enzymatic treatment of said population of antibodies with galactosyltransferase to obtain an antibody population, which comprises a relative frequency of at least 70 % of Fc bi-galactosylated antibodies and subsequent separation of the population of antibodies from said enzyme.

Another aspect of the invention is the use of said method for improving ADCC mediated by said population of recombinant antibodies of IgG1 isotype.

Another aspect of the invention is the population of galactoengineered recombinant antibodies of IgG1 isotype according to the invention for mediation of ADCC.

Another aspect of the invention is the population of galactoengineered recombinant antibodies of IgG1 isotype according to the invention for use as a medicament.

According to the invention the effector functions, specifically ADCC mediation, of IgG1 molecules are tailored by means of galactoengineering. The invention provides means and methods to adjust the glycan profile of recombinant IgG1 after their production in order to provide a substantially homogeneous glycan pattern, which is reproducible between different batches of the generated IgG1 antibody. The invention further allows improvement of ADCC mediated by the IgG1 molecules by providing IgG1 molecules with a certain glycan profile by glycoengineering.

### DESCRIPTION OF THE FIGURES

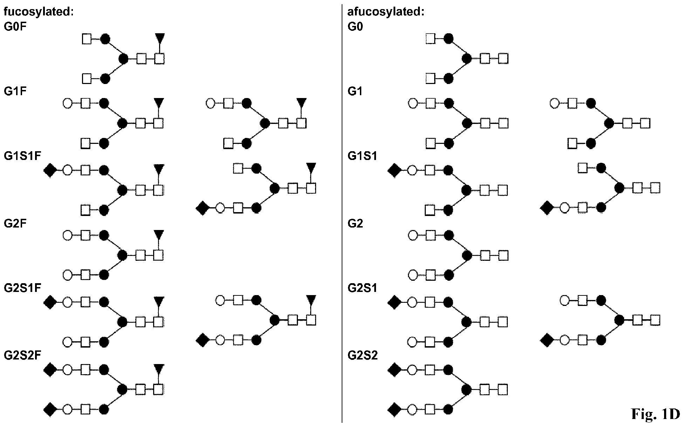

- **Figure 1:**: Schematic illustration of N-linked glycans of IgG molecules (legend of symbols used within the illustration: white square - N-acetylglucosamine, black circle - mannose, black triangle - fucose, white circle - galactose, black diamond - sialic acid). Sialic acid of recombinantly expressed IgG molecules typically is 5-N-acetylneuraminic acid (NANA) or 5-N-glycolylneuraminic acid (NGNA), while human IgG molecules only comprise NANA.
**Figure 1A****:** Exemplary high mannose type glycan structure, including two N-acetylglucosamines with many mannose residues;
**Figure 1B****:** Exemplary hybrid glycan structure, comprising a branch of mannose residues and a complex branch;
**Figure 1C****:** Exemplary complex glycan structure, indicated are all possibly attached sugar residues including bisecting N-acetylglucosamine;
**Figure 1D****:** Fucosylated and afucosylated complex biantennary structures and the corresponding abbreviations as used herein.

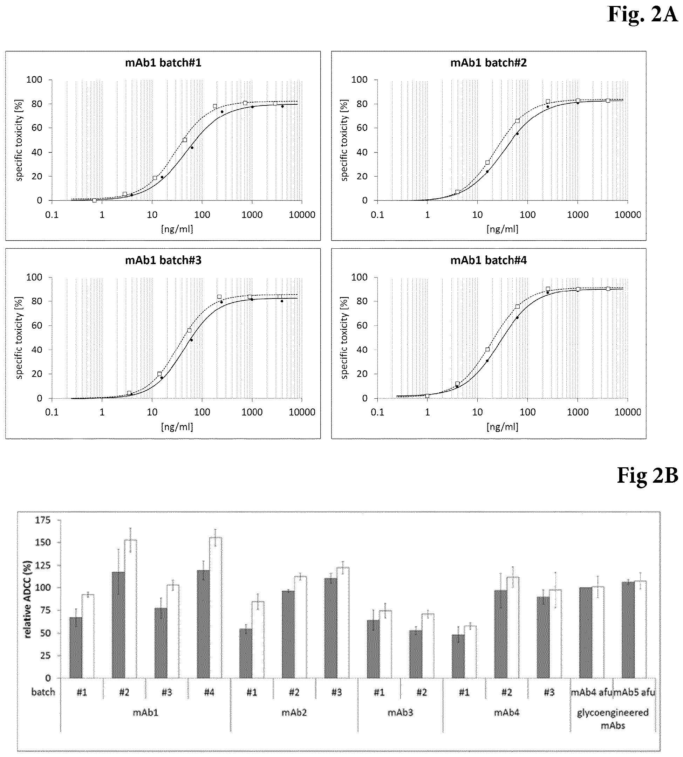

- **Figure 2:**: ADCC of enzymatically hypergalactosylated antibodies from different production batches (#1 - #4)
**Figure 2A****:** Exemplary dose dependent ADCC mediated by untreated trastuzumab (black circles, solid lines) and enzymatically hypergalactosylated trastuzumab (white squares, dashed lines) from the respective production batches as indicated;
**Figure 2B****:** Comparison of ADCC mediated by untreated control (grey bars) and enzymatically hypergalactosylated material (white bars). Error bars represent standard deviations between individual experiments (n ≥ 3). mAb1 = Trastuzumab; mAb2 = Rituximab; mAb3 = Pertuzumab; mAb5 = Obinutuzumab; afu = afucosylated.

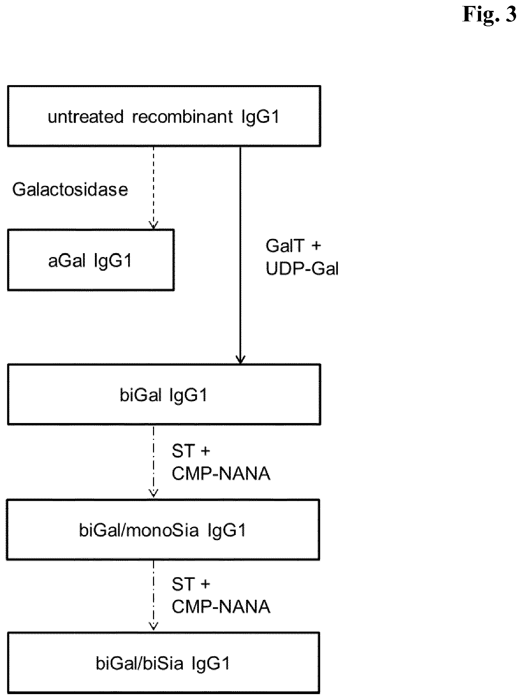

- **Figure 3:**: Schematic workflow of *in vitro* galactoengineering process used for sample preparation in Example 3. Recombinant IgG1 was produced in CHO cells and purified (untreated recombinant IgG1). To prepare an agalactosylated (aGal) control sample, the antibodies were treated with galactosidase. To prepare a population of antibodies with a high relative frequency of bi-galactosylated IgG1 (biGal IgG1), the untreated antibodies were treated with galactosyltransferase by a method according to the invention as described in example 3. To provide a population of IgG1 with a high relative frequency of monosialylated IgG1 (biGal/monoSia IgG1), the bi-galactosylated antibodies were treated with sialyltransferase as described for a method according to the invention and in example 3. To generate a population of antibodies with a high relative frequency of bi-sialylated IgG1 (biGal/biSia) the sialylated population of antibodies was subjected to another sialyltransferase treatment as described in example 3.

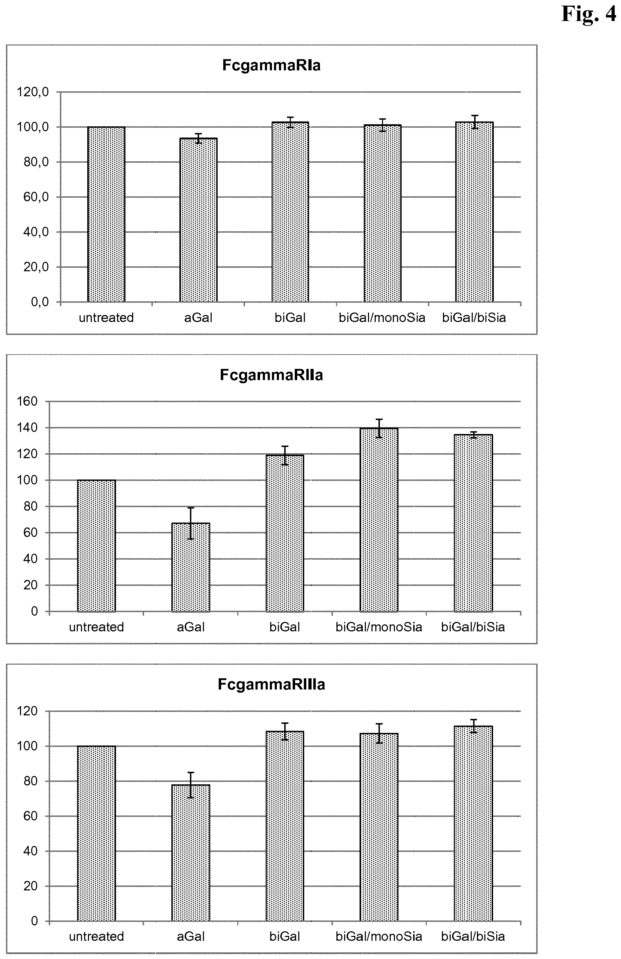

- **Figure 4:**: Fc-gamma-R binding as analyzed by surface plasmon resonance. Indicated is the binding of the different IgG1 antibody populations of example 3 (including untreated control sample and agalactosylated comparative samples) to Fc-gammaRIa, Fc-gammaRIIa and Fc-gammaRIIIa as analyzed by SPR relative to the binding of the untreated population of antibodies (which is set to 100 %). All samples were analyzed in triplicate.

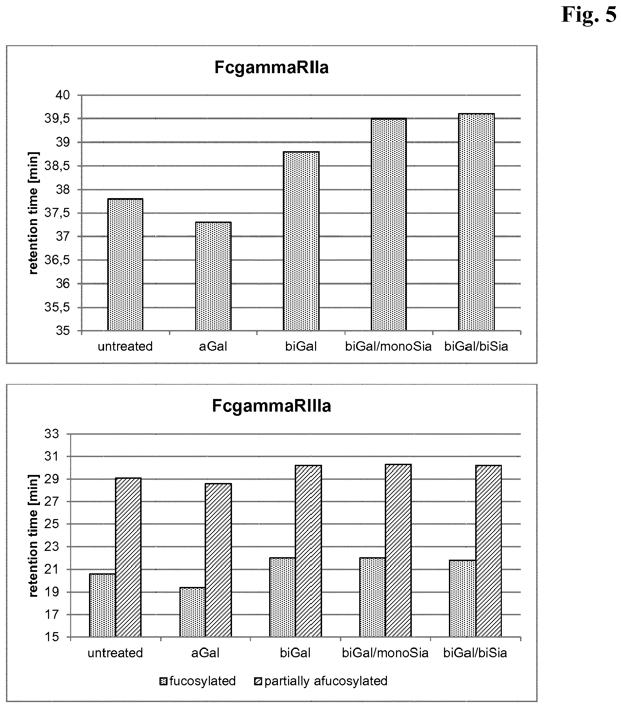

- **Figure 5:**: Fc-gamma-R binding as analyzed by affinity chromatography. Indicated is the binding of the different IgG1 antibody populations of example 3 (including untreated control sample and agalactosylated comparative samples) to Fc-gammaRIIa and Fc-gammaRIIIa as analyzed by affinity chromatography.

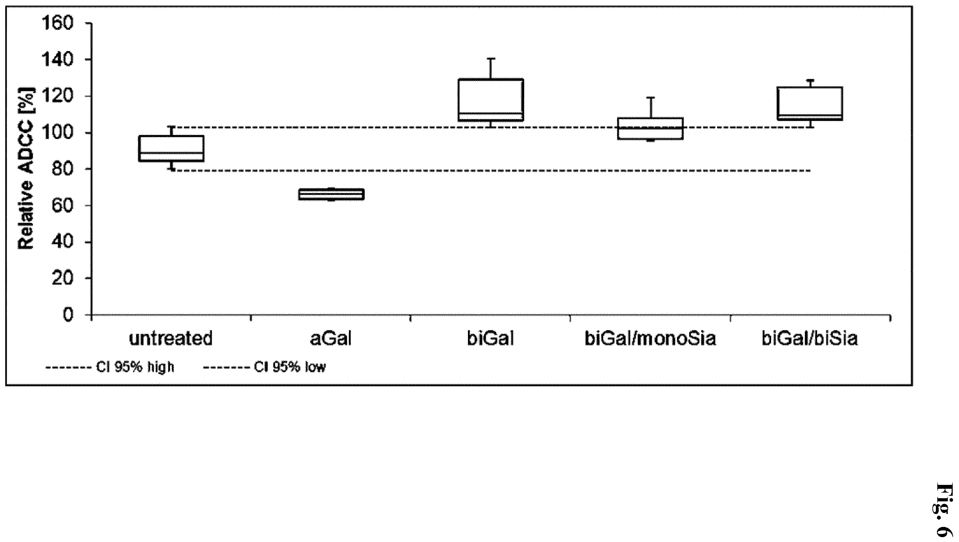

- **Figure 6:**: ADCC of IgG1 antibodies of different glycan species. Indicated is the relative ADCC as analyzed by the cell based ADCC assay as described in examples 2 and 7 for the different IgG1 antibody populations of example 3 (including untreated control sample and agalactosylated comparative samples). Indicated is the untreated control IgG1 population as well as enzyme treated populations aGal (comparative example), biGal, biGal/monoSia and biGal/biSia.

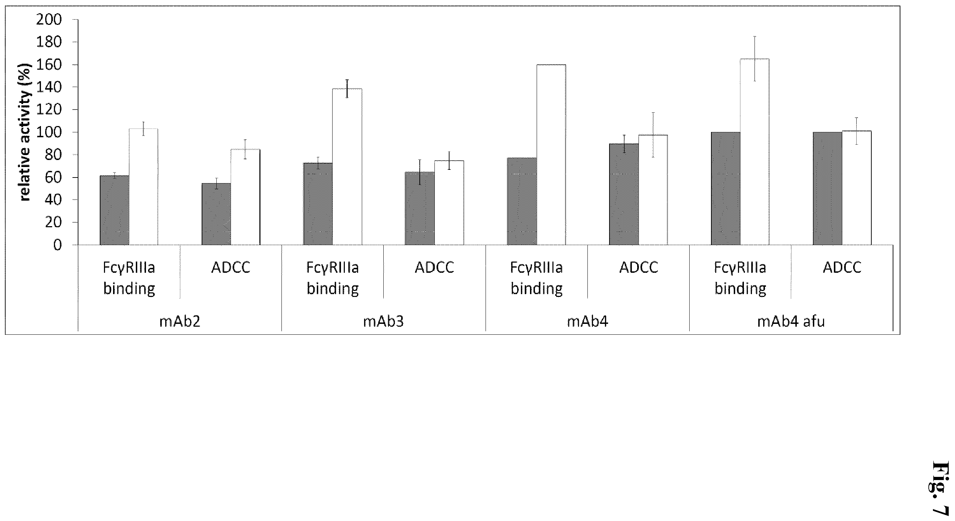

- **Figure 7:**: Effect of hypergalactosylation on Fc-gammaRIIIa binding and ADCC. Fc-gammaRIIIa binding and ADCC relative to reference material; untreated control (grey bars), hypergalactosylated material (white bars). Error bars represent standard deviations between independent experiments (n ≥ 3). mAb2 = Rituximab; mAb3 = Pertuzumab; afu = afucosylated.

### DETAILED DESCRIPTION OF THE INVENTION

### 1. Definitions

The terms "a", "an" and "the" generally include plural referents, unless the context clearly indicates otherwise.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

The terms "full length antibody", "intact antibody", and "whole antibody" are used herein interchangeably to refer to an antibody having a structure substantially similar to a native antibody structure or having heavy chains that contain an Fc region as defined herein.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

An "isolated" antibody is one which has been separated from a component of its natural environment. In some embodiments, an antibody is purified to greater than 95 % or 99 % purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called a, d, e, g, and m, respectively. Antibodies according to the invention are of IgG1 isotype.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

"Antibody-dependent cell-mediated cytotoxicity" or "ADCC" refers to a form of cytotoxicity in which secreted immunoglobulins (Ig) bound onto Fc receptors (FcRs) present on certain cytotoxic cells (e.g. NK cells, neutrophils, and macrophages) enable these cytotoxic effector cells to bind specifically to an antigen-bearing target cell and subsequently kill the target cell with cytotoxins. The primary cells for mediating ADCC, NK cells, express Fc-gammaRIII only, whereas monocytes express Fc-gammaRI, Fc-gammaRII, and Fc-gammaRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed *in vivo,* e.g., in an animal model such as that disclosed in Clynes et al. PNAS (USA) 95:652-656 (1998).

"Complement dependent cytotoxicity" or "CDC" refers to the lysis of a target cell in the presence of complement. Activation of the classical complement pathway is initiated by the binding of the first component of the complement system (Clq) to antibodies (of the appropriate subclass), which are bound to their cognate antigen. To assess complement activation, a CDC assay, e.g., as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, e.g., in US Patent No. 6,194,551 B1 and WO 1999/51642. See also, e.g., Idusogie et al. J. Immunol. 164: 4178-4184 (2000).

"Fc receptor" or "FcR" describes a receptor that binds to the Fc region of an antibody. In some embodiments, an FcR is a native human FcR. In some embodiments, an FcR is one which binds an IgG antibody (a gamma receptor) and includes receptors of the Fc-gammaRI, Fc-gammaRII, and Fc-gammaRIII subclasses, including allelic variants and alternatively spliced forms of those receptors. Fc-gammaRII receptors include Fc-gammaRIIA (an "activating receptor") and Fc-gammaRIIB (an "inhibiting receptor"), which have similar amino acid sequences that differ primarily in the cytoplasmic domains thereof. Activating receptor Fc-gammaRIIA contains an immunoreceptor tyrosine-based activation motif (ITAM) in its cytoplasmic domain. Inhibiting receptor Fc-gammaRIIB contains an immunoreceptor tyrosine-based inhibition motif (ITIM) in its cytoplasmic domain (see, e.g., Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

An "activating Fc receptor" is an Fc receptor that following engagement by an Fc region of an antibody (or immunoconjugate) elicits signaling events that stimulate the receptor-bearing cell to perform effector functions. Activating Fc receptors include Fc-gammaRIIIa (CD16a), Fc-gammaRI (CD64), Fc-gammaRIIa (CD32), and FcaRI (CD89).

A "mode of action" of an IgG1 antibody describes a functional or anatomical change, at the cellular level, resulting from the exposure of a living organism to said antibody. Antibodies comprising an IgG1 Fc region efficiently activate the immune system. Therapeutic application of IgG1 thereby performed with the scope of harnessing different immune cells and molecules towards tumor target cell killing. Antibodies of IgG1 isotype activate NK cells through CD16A and induce antibody dependent cytotoxicity (ADCC). Thereby, one mode of action of IgG1 is induction of ADCC. Antibodies of IgG1 isotype activate complement leading to complement dependent cytotoxicity (CDC). Thereby, another mode of action of IgG1 is induction of CDC.

A "recombinant antibody" is an antibody which has been produced by a recombinantly engineered host cell. It is optionally isolated or purified.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "chimeric" antibody refers to an antibody in which a portion of the heavy and/or light chain is derived from a particular source or species, while the remainder of the heavy and/or light chain is derived from a different source or species. A "humanized" antibody refers to a chimeric antibody comprising amino acid residues from non-human HVRs and amino acid residues from human frameworks (FRs). In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

IgG antibodies are glycoproteins comprising a conserved Fc glycosylation site at Asn297 in the CH2 domain of the heavy chain constant regions. The term "glycosylation site" refers to an amino acid within the amino acid sequence of the antibody to which a glycan is covalently linked. N-linked glycans are typically linked to the nitrogen atom of an asparagine (Asn) side chain that is present as a part of an Asn-X-Ser or an Asn-X-Thr consensus sequence, wherein X is an amino acid except proline, Ser is serine and Thr is threonine. While the conserved glycosylation site in the Fc region is present in all wild type IgG antibodies, further glycosylation sites may be present within the Fab region of the antibodies.

"Asn297" as used herein means the amino acid asparagine located at about position 297 in the Fc region of IgG1 molecules. Based on minor sequence variations of antibodies, Asn297 can also be located some amino acids (usually not more than +3 amino acids) upstream or downstream of position 297, i.e. between position 294 and 300.

The term "N-linked glycan" refers to an oligosaccharide covalently linked to the nitrogen atom of an asparagine residue of the antibody. In general, three major types of N-linked glycans are known, including high-mannose structures (Fig. 1A), complex biantennary structures (Fig. 1C) and hybrid structures (Fig. 1B) with a branch of mannose residues and a complex branch. The N-linked glycans of Asn297 of the CH2 domains are complex biantennary oligosaccharides, which are buried between the CH2 domains, forming extensive contacts with the polypeptide backbone. Their presence is essential for the antibody to mediate effector functions such as antibody dependent cellular cytotoxicity (ADCC) (Lifely, M., R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32).

A "glycosylation variant" refers to an antibody in which any carbohydrate attached to the Fc region is altered. Hence, of the population of antibodies according to the invention includes glycosylation variants of the antibodies that were recombinantly expressed by the host cell.

Different complex biantennary structures of N-linked glycans are indicated in Fig. ID. The term "galactosylated" antibody as used herein refers to an antibody, wherein the N-linked glycan of the antibody (in one embodiment the N-linked glycan coupled to Asn297 of the antibody) comprises at least one galactose residue (including G1F, G1, G2F, G2, G1S1F, G1S1, G2S1F, G2S1, G2S2F and G2S2 structures). The term "bi-galactosylated" antibody as used herein refers to an antibody, wherein the N-linked glycan of the antibody (in one embodiment the N-linked glycan coupled to Asn297 of the antibody) is a complex biantennary structure comprising exactly two galactose residues (including G2F, G2, G2S1F, G2S1, G2S2F and G2S2 structures). The term "mono-galactosylated" antibody as used herein refers to an antibody, wherein the N-linked glycan of the antibody (in one embodiment the N-linked glycan coupled to Asn297 of the antibody) is a complex biantennary structure comprising exactly one galactose residue (including G1F, G1, G1S1F and G1S1 structures). The term "agalactosylated" antibody as used herein refers to an antibody, wherein the N-linked glycan of the antibody (in one embodiment the N-linked glycan coupled to Asn297 of the antibody) is devoid of a galactose residue (including G0, G0F and further structures devoid of a galactose residue).

The term "sialylated" antibody as used herein refers to an antibody, wherein the N-linked glycan of the antibody (in one embodiment the N-linked glycan coupled to Asn297 of the antibody) comprises at least one sialic acid residue (including G1S1F, G1S1, G2S1F, G2S1, G2S2F and G2S2 structures). The term "sialic acid" as used herein refers to an N-substituted derivative of neuraminic acid, particularly including 5-N-acetylneuraminic acid (NANA) or 5-N-glycolylneuraminic acid (NGNA). While human IgG molecules only comprise sialylated N-linked glycans with NANA as sialic acid, recombinantly expressed IgG molecules (i.e. in host cells other than human cells) comprise sialylated N-linked glycans with both NANA or NGNA as sialic acid. The term "bi-sialylated" antibody as used herein refers to an antibody, wherein the N-linked glycan of the antibody (in one embodiment the N-linked glycan coupled to Asn297 of the antibody) is a complex biantennary structure comprising exactly two sialic acid residues (including G2S2F and G2S2 structures). The term "mono-sialylated" antibody as used herein refers to an antibody, wherein the N-linked glycan of the antibody (in one embodiment the N-linked glycan coupled to Asn297 of the antibody) is a complex biantennary structure comprising exactly one sialic acid residue (including G1S1F, G1S1, G2S1F and G2S1 structures). The term "asialylated" antibody as used herein refers to an antibody, wherein the N-linked glycan of the antibody (in one embodiment the N-linked glycan coupled to Asn297 of the antibody) is devoid of a sialic acid residue (including G0, G0F and further structures devoid of a sialic acid residue).

The term "fucosylated" antibody as used herein refers to an antibody, wherein the N-linked glycan of the antibody (in one embodiment the N-linked glycan coupled to Asn297 of the antibody) comprises a fucose residue (including G1F, G2F, G1S1F, G2S1F, G2S2F and further structures comprising a fucose residue). The term "afucosylated" antibody as used herein refers to an antibody, wherein the N-linked glycan of the antibody (in one embodiment the N-linked glycan coupled to Asn297 of the antibody) is devoid of a fucose residue (including G1, G2, G1S1, G2S1, G2S2 and further structures devoid of a fucose residue). The term "afucosylated" is herein also abbreviated as "afu".

The term "devoid of' a distinct sugar residue as used herein means that the glycan pattern of neither one of the CH2 domains of an antibody comprises said distinct sugar residue. Hence, "devoid of galactose" means that the glycan pattern of neither one of the CH2 domains of an antibody comprises a galactose residue. Accordingly, "devoid of fucose" means that the glycan pattern of neither one of the CH2 domains of an antibody comprises a fucose residue.

When referring to antibodies that are "substantially devoid of' a distinct sugar residue (e.g. galactose, fucose or sialic acid) as used herein, it is meant that the population of antibodies comprises only a minor relative frequency of antibodies comprising said distinct sugar residue such that one of skill in the art would consider the fraction of antibodies comprising said distinct sugar residue of little or no biological significance within the context of the biological characteristics of the antibody (e.g. ADCC mediated by the antibody, FcR binding). Hence, antibodies that are "substantially devoid of Fc galactose residues" means a population of antibodies that comprises only a minor relative frequency of antibodies comprising galactose residues such that one of skill in the art would consider the fraction of antibodies comprising said distinct sugar residue of little or no biological significance within the context of the biological characteristics of the antibody (e.g. ADCC mediated by the antibody, FcR binding). Antibodies that are "substantially devoid of' a distinct sugar residue (e.g. galactose, fucose or sialic acid) may be present in a population of antibodies with a relative frequency of 2 % or less, preferably 1 % or less, preferably 0.5 % or less. Accordingly, a population of antibodies that are substantially devoid of a distinct sugar residue comprises a relative frequency of at least 98 %, preferably at least 99 %, preferably at least 99.5 % antibodies devoid of said distinct sugar residue.

By the term "population of antibodies" is meant a mixture of, preferably monoclonal, antibodies that may comprise a heterogeneous glycan pattern. In particular, the antibodies may exhibit heterogeneity with respect to the N-linked glycan pattern of their Fc region. IgG antibodies expressed in recombinant expression systems, e.g. by expression in prokaryotic or eukaryotic host cells, comprise a heterogeneous N-linked glycan structure that may vary between individual antibody molecules and between different batches of generated antibodies. Within the population of antibodies, the individual antibodies are substantially identical in their amino acid sequence, except for possible variant antibodies, e.g., containing naturally occurring mutations or mutations arising during production of a monoclonal antibody preparation, such variants generally being present in minor amounts.

The "relative frequency" of a population of antibodies as referred to herein means the percentage of a distinct glycan pattern within the population of antibodies related to all glycostructures within the population of antibodies. In particular, the relative frequency of a population of antibodies refers to the percentage of a distinct glycan pattern within the population of antibodies related to all glycostructures identified in an PNGase F treated sample of the population of antibodies that was analyzed by NP-UHPLC. For example, a population of antibodies comprising a relative frequency of 70 % of bi-galactosylated antibodies means herein that 70 % of the glycostructures within the population of antibodies are complex biantennary structures comprising exactly two galactose residues (including G2F, G2, G2S1F, G2S1, G2S2F and G2S2 structures), wherein the remaining 30 % of all glycostructures within the population of antibodies are i) glycostructures with a complex biantennary structure comprising exactly one galactose residue (including G1F, G1, G1S1F and G1S1 structures, and/or ii) glycostructures with a complex biantennary structure but devoid of a galactose residue (including G0, G0F and further structures devoid of a galactose residue).

The term *"in vitro* glycoengineering" as referred to herein means the alteration of the N-linked glycan structure of antibodies that is performed *in vitro* after the antibodies are expressed in a recombinant expressions system and, preferably, purified. *In vitro* glycoengineering within the terms of the invention always encompasses the addition of at least one sugar residue to the N-linked glycan structure of at least a fraction of the antibodies comprised within the population of antibodies that was subject of *in vitro* glycoengineering. Optionally, *in vitro* glycoengineering may encompass the cleavage of at least one sugar residue from the N-linked glycan structure of at least a fraction of the antibodies comprised within the population of antibodies that was subject of *in vitro* glycoengineering. Within the scope of the invention, the term *"in vitro* glycoengineering" does not encompass, i.e. specifically excludes, the addition or cleavage of a fucose residue from the from the N-linked glycan structure of antibodies.

*In vitro* glycoengineering within the terms of the invention includes at least one step of enzymatic treatment of antibodies that were expressed in a recombinant expressions system and, preferably, purified. In one embodiment said enzymatic treatment includes treatment of the antibodies with a glycosyltransferase, preferably a glycosyltransferase capable of adding a terminal sugar residue to an N-linked oligosaccharide structure of a protein. In one embodiment said enzymatic treatment includes treatment of the antibodies with a galactosyltransferase, preferably beta-1,4-galactosyltransferase. *In vitro* glycoengineering within the terms of the invention may additionally include at least one step of enzymatic treatment with an enzyme capable of cleaving a terminal sugar residue from an N-linked oligosaccharide structure of a protein. In one embodiment, said enzyme is selected from a galactosidase and a neuraminidase. In addition, *in vitro* glycoengineering within the terms of the invention may additionally include at least one step of enzymatic treatment with a sialyltransferase, preferably sialyltransferase 6 (ST6, catalyzing the formation of alpha-2,6-glycosidic bonds) or sialyltransferase 3 (ST3, catalyzing the formation of alpha-2,3-glycosidic bonds).

Glycoengineering can also be performed *in vivo,* e.g. by overexpressing in a cell line an enzyme capable of adding a terminal sugar residue to or cleaving a terminal sugar residue from an N-linked oligosaccharide structure of a protein. Furthermore, glycoengineering can also be performed *in vivo,* e.g. by reducing the expression of or knocking out an enzyme in a cell line, the enzyme being capable of adding a terminal sugar residue to or cleaving a terminal sugar residue from an N-linked oligosaccharide structure of a protein.

The term "galactoengineering" as referred to herein means the alteration of the N-linked glycan structure of antibodies related to the galactose residues only, i.e. the alteration of the number of galactose residues within the N-linked glycan structure of antibodies. Galactoengineering within the terms of the invention always encompasses the addition of at least one galactose residue to the N-linked glycan structure of at least a fraction of the antibodies comprised within the population of antibodies that was subject of galactoengineering. Optionally, galactoengineering may encompass the cleavage of at least one galactose residue from the N-linked glycan structure of at least a fraction of the antibodies comprised within the population of antibodies that was subject of galactoengineering. Galactoengineering can be performed *in vitro.* Galactoengineering can also be performed *in vivo,* e.g. by i) overexpressing in a cell line an enzyme capable of adding a terminal galactose residue to or cleaving a terminal galactose residue from an N-linked oligosaccharide structure of a protein, or ii) by reducing the expression or by knock-out of an enzyme in a cell line, the enzyme being capable of adding a terminal galactose residue to or cleaving a terminal galactose residue from an N-linked oligosaccharide structure of a protein. As used herein, "galactoengineering" is a specific form of "glycoengineering" and "galactoengineered" is a specific form of "glycoengineered".

The term *"in vitro* galactoengineering" within the terms of the invention includes at least one step of enzymatic treatment of antibodies that were expressed in a recombinant expressions system and, preferably, purified. In one embodiment said enzymatic treatment includes treatment of the antibodies with a galactosyltransferase, preferably a galactosyltransferase capable of adding a terminal galactose residue to an N-linked oligosaccharide structure of a protein. In one embodiment said enzymatic treatment includes treatment of the antibodies with beta-1,4-galactosyltransferase. *In vitro* galactoengineering within the terms of the invention may additionally include at least one step of enzymatic treatment with an enzyme capable of cleaving a terminal galactose residue from an N-linked oligosaccharide structure of a protein. In one embodiment, said enzyme is a galactosidase.

Antibodies according to the invention are produced by recombinant means. Methods for recombinant production of antibodies are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective antibody light and heavy chains are inserted into expression vectors by standard methods. Expression is performed in appropriate prokaryotic or eukaryotic host cells, like CHO cells, NS0 cells, SP2/0 cells, HEK293 cells, COS cells, PER.C6 cells, yeast, or E. coli cells, and the antibody is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR2 ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a vector capable of directing the expression of nucleic acids to which they are operatively linked. When the expression vector is introduced into an appropriate host cell, it can be transcribed and translated into a polypeptide. When transforming host cells in methods according to the invention, "expression vectors" are used; thereby the term "vector" in connection with transformation of host cells as described herein means "expression vector". An "expression system" usually refers to a suitable host cell comprised of an expression vector that can function to yield a desired expression product.

As used herein, "expression" refers to the process by which a nucleic acid is transcribed into mRNA and/or to the process by which the transcribed mRNA (also referred to as a transcript) is subsequently translated into a peptide, polypeptide, or protein. The transcripts and the encoded polypeptides are individually or collectively referred to as gene products. If a nucleic acid is derived from genomic DNA, expression in a eukaryotic cell may include splicing of the corresponding mRNA.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen, F.N, et al., PNAS 69 (1972) 7110 et seq.

The term "host cell" as used in the current application denotes any kind of cellular system which can be engineered to generate the antibodies according to the current invention.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., J. Immunol. Methods 194 (1996) 191-199.

The term "purified" as used herein refers to polypeptides, that are removed from their natural environment or from a source of recombinant production, or otherwise isolated or separated, and are at least 60 %, e.g., at least 80 %, free from other components, e.g. membranes and microsomes, with which they are naturally associated. Purification of antibodies (recovering the antibodies from the host cell culture) is performed in order to eliminate cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. To administer an antibody according to the invention by certain routes of administration, it may be necessary to coat the antibody with, or co-administer the antibody with, a material to prevent its inactivation. For example, the antibody may be administered to a subject in an appropriate carrier, for example, liposomes, or a diluent. Pharmaceutically acceptable diluents include saline and aqueous buffer solutions.

A pharmaceutical composition comprises an effective amount of the antibodies according to the invention. An "effective amount" of an agent, e.g., an antibody, refers to an amount effective, at dosages and for periods of time necessary, to achieve the desired therapeutic or prophylactic result. In particular, the "effective amount" denotes an amount of an antibody of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the antibody molecules used, disease state being treated, the severity or the disease treated, the age and relative health of the subject, the route and form of administration, the judgement of the attending medical or veterinary practitioner, and other factors.

A "pharmaceutically acceptable carrier" refers to an ingredient in a pharmaceutical formulation, other than an active ingredient, which is nontoxic to a subject. Pharmaceutically acceptable carriers includes any and all solvents, dispersion media, coatings, antibacterial and antifungal agents, isotonic and absorption delaying agents, and the like that are physiologically compatible. In one embodiment, the carrier is suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, in one embodiment the carrier is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

HER2 is a member of the human epidermal growth factor receptor family and possesses protein kinase activity in its cytoplasmic domain. HER2 is over-expressed in tumor cells and is correlated with poor prognosis and survival. HER2 is therefore a valuable target of breast cancer therapy. Antibodies against HER2 are known from Takai, N., et al., Cancer 104 (2005) 2701-2708; Yeon, C. H., et al., Invest. New Drugs 23 (2005) 391-409; Wong, W. M., et al., Cancer Pract. 7 (1999) 48-50; Albanell, J., et al., Drugs Today (Barc). 35 (1999) 931-46.

The CD20 antigen is a ∼35 kDa, non-glycosylated phosphoprotein found on the surface of greater than 90 % of B cells from peripheral blood or lymphoid organs. CD20 is expressed during early pre-B cell development and remains until plasma cell differentiation. CD20 is present on both normal B cells as well as malignant B cells. Other names for CD20 in the literature include "B-lymphocyte-restricted antigen" and "Bp35". The CD20 antigen is described in Clark et al. PNAS (USA) 82:1766 (1985), for example.

Trastuzumab (INN), also referred herein as to mAb1, is a recombinant humanized anti-HER2 monoclonal antibody used for the treatment of HER2 over-expressed/HER2 gene amplified metastatic breast cancer. Preclinical studies demonstrated that the antibody has anti-tumor activity *in vivo* and *in vitro.* Moreover, additive or synergistic enhancement of anti-tumor activity of trastuzumab was observed in combination with various anti-tumor agents in mouse models. In clinical studies, extension of survival was observed in HER2 overexpressing metastatic breast cancer patients. Trastuzumab is reported in WO 92/022653, WO 99/057134, WO 97/04801, US 5,677,171 and US 5,821,337 (incorporated herein by reference).

Rituximab (INN), also referred herein as to mAb2, is a genetically engineered chimeric murine/human monoclonal antibody directed against the CD20 antigen and designated "C2B8" in U.S. Pat. No. 5,736,137, expressly incorporated herein by reference. The antibody is an IgG1 kappa immunoglobulin containing murine light and heavy chain variable region sequences and human constant region sequences.

Pertuzumab (INN), also referred herein as to mAb3, is a recombinant humanized monoclonal antibody, generated based on human IgG1(κ) framework. Pertuzumab was developed as a humanized antibody that inhibits the dimerization of HER2 with other HER receptors, thereby inhibiting ligand-driven phosphorylation and activation, and downstream activation of the RAS and AKT pathways.

Obinutuzumab (INN, formerly known as afutuzumab), also referred herein as to mAb5, is a humanized IgG1 antibody directed against the CD20 antigen.

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "inflammatory disease" and "inflammatory disorder" are used interchangeably and mean a disease or disorder in which a component of the immune system of a mammal causes, mediates or otherwise contributes to an inflammatory response contributing to morbidity in the mammal. Also included are diseases in which reduction of the inflammatory response has an ameliorative effect on progression of the disease. Included within this term are immune-mediated inflammatory diseases, including autoimmune diseases.

An "autoimmune disorder" herein is a disease or disorder arising from and directed against an individual's own tissues or organs or a co-segregation or manifestation thereof or resulting condition therefrom. In many of these autoimmune and inflammatory disorders, a number of clinical and laboratory markers may exist, including, but not limited to, hypergammaglobulinemia, high levels of autoantibodies, antigen-antibody complex deposits in tissues, benefit from corticosteroid or immunosuppressive treatments, and lymphoid cell aggregates in affected tissues. Without being limited to any one theory regarding B-cell mediated autoimmune disorder, it is believed that B cells demonstrate a pathogenic effect in human autoimmune diseases through a multitude of mechanistic pathways, including autoantibody production, immune complex formation, dendritic and T-cell activation, cytokine synthesis, direct chemokine release, and providing a nidus for ectopic neo-lymphogenesis. Each of these pathways may participate to different degrees in the pathology of autoimmune diseases.

"Autoimmune disease" can be an organ-specific disease (i.e., the immune response is specifically directed against an organ system such as the endocrine system, the hematopoietic system, the skin, the cardiopulmonary system, the gastrointestinal and liver systems, the renal system, the thyroid, the ears, the neuromuscular system, the central nervous system, etc.) or a systemic disease that can affect multiple organ systems (for example, systemic lupus erythematosus (SLE), rheumatoid arthritis (RA), polymyositis, etc.). Preferred such diseases include autoimmune rheumatologic disorders (such as, for example, RA, Sjögren's syndrome, scleroderma, lupus such as SLE and lupus nephritis, polymyositis-dermatomyositis, cryoglobulinemia, anti-phospholipid antibody syndrome, and psoriatic arthritis), autoimmune gastrointestinal and liver disorders (such as, for example, inflammatory bowel diseases (e.g., ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-negative vasculitis and ANCA-associated vasculitis, including Churg-Strauss vasculitis, Wegener's granulomatosis, and microscopic polyangiitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g., Graves' disease and thyroiditis)). More preferred such diseases include, for example, RA, ulcerative colitis, ANCA-associated vasculitis, lupus, multiple sclerosis, Sjögren's syndrome, Graves' disease, IDDM, pernicious anemia, thyroiditis, and glomerulonephritis.

### 2. Detailed description of the embodiments of the invention

The invention provides recombinant IgG1 antibodies with an altered N-linked glycan profile that are glycoengineered by an enzymatic treatment to comprise a high amount of terminal galactose residues and optionally sialic acid residues and thereby, exhibit an improved capability to mediate ADCC. Further, the invention provides glycoengineering methods for generating said antibodies. By means of glycoengineering a population with a substantially homogeneous glycan pattern can be generated, which can be reproducibly created for antibodies produced in different expression batches of an individual type of host cell. With the invention it was demonstrated that IgG1 antibodies exhibiting a distinct balance between N-linked galactosylated and fucosylated glycan structures show improved ADCC properties. The ADCC properties of said antibodies can be adjusted by adding (terminal) galactose residues by means of galactoengineering. Addition of terminal sialic acid residues via glycoengineering preserves the ADCC properties but in addition may improve the biological properties of the antibody (e.g. increase of serum half-life).

### a) Antibodies

In one aspect the invention relates to a population of galactoengineered recombinant antibodies of IgG1 isotype, comprising a relative frequency of at least 80 % of Fc galactosylated antibodies.

In one aspect the invention relates to a population of galactoengineered recombinant antibodies of IgG1 isotype, comprising a relative frequency of at least 70 % of Fc bi-galactosylated antibodies.

In one embodiment of the invention, the population of antibodies comprises a relative frequency of less than 20 % of Fc afucosylated antibodies.

Hence, in one embodiment the invention relates to a population of galactoengineered recombinant antibodies of IgG1 isotype, comprising a relative frequency of at least 80 % of Fc galactosylated antibodies and a relative frequency of less than 20 % of Fc afucosylated antibodies. In another embodiment the invention relates to a population of *in vitro* glycoengineered recombinant antibodies of IgG1 isotype, comprising a relative frequency of at least 70 % of Fc bi-galactosylated antibodies and a relative frequency of less than 20 % of Fc afucosylated antibodies.

In one embodiment of the invention the population of antibodies comprises a relative frequency of up to 100 % of Fc galactosylated antibodies (including mono-galactosylated and bi-galactosylated glycan structures). In one embodiment, the population of antibodies comprises a relative frequency of up to 99.5 % of Fc galactosylated antibodies. In one embodiment, the population of antibodies comprises a relative frequency of up to 95 % of Fc galactosylated antibodies. In one embodiment, the population of antibodies comprises a relative frequency of up to 90 % of Fc galactosylated antibodies. In one embodiment, the population of antibodies comprises a relative frequency of 80 % to 99.5 % of Fc galactosylated antibodies.

In one embodiment of the invention the population of antibodies comprises a relative frequency of up to 98 % of Fc bi-galactosylated antibodies. In one embodiment, the population of antibodies comprises a relative frequency of up to 95 % of Fc bi-galactosylated antibodies. In one embodiment, the population of antibodies comprises a relative frequency of up to 90 % of Fc bi-galactosylated antibodies. In one embodiment, the population of antibodies comprises a relative frequency of 70 % to 95 % of Fc bi-galactosylated antibodies. In one embodiment, the population of antibodies comprises a relative frequency of 80 % to 95 % of Fc bi-galactosylated antibodies. In one embodiment, the population of antibodies comprises a relative frequency of 80 % to 90 % of Fc bi-galactosylated antibodies.

In one embodiment of the invention, the population of antibodies comprises a relative frequency of up to 16 % of Fc afucosylated antibodies. In one embodiment of the invention the population of antibodies comprises a relative frequency of at least 2 % of Fc afucosylated antibodies. In one embodiment, the population of antibodies comprises a relative frequency of at least 5 % of Fc afucosylated antibodies. In one embodiment, the population of antibodies comprises a relative frequency of at least 7 % of Fc afucosylated antibodies. In one embodiment, the population of antibodies comprises a relative frequency of 2 - 20 % of Fc afucosylated antibodies. In one embodiment, the population of antibodies comprises a relative frequency of 5 - 20 % of Fc afucosylated antibodies. In one embodiment, the population of antibodies comprises a relative frequency of 7 - 16 % of Fc afucosylated antibodies. In one embodiment, the population of antibodies comprises a relative frequency of 8 - 12 % of Fc afucosylated antibodies.

In one embodiment of the invention the population of antibodies has not undergone glycoengineering to improve afucosylation. In one embodiment, the population of antibodies comprises a relative frequency of Fc afucosylated antibodies corresponding to the relative frequency of Fc afucosylation in antibodies expressed in the same host cell. In one embodiment, the population of antibodies comprises a relative frequency of Fc afucosylated antibodies corresponding to the relative frequency of Fc afucosylation in antibodies expressed in a CHO cell.

In one embodiment of the invention the population of antibodies comprises a relative frequency of up to 98 % of Fc sialylated antibodies (including mono-sialylated and bi-sialylated glycan structures). In one embodiment, the population of antibodies comprises a relative frequency of up to 97 % of Fc sialylated antibodies. In one embodiment, the population of antibodies comprises a relative frequency of up to 95 % of Fc sialylated antibodies. In one embodiment, the population of antibodies comprises a relative frequency of up to 90 % of Fc sialylated antibodies. In one embodiment, the population of antibodies comprises a relative frequency of 50 % to 90 % of Fc sialylated antibodies. In one embodiment, the population of antibodies comprises a relative frequency of 50 % to 80 % of Fc sialylated antibodies.

In one embodiment, the sialylated antibody comprises NANA or NGNA as sialic acid. In one embodiment, the sialylated antibody comprises NANA as sialic acid. In one embodiment, the sialylated antibody comprises NANA as sialic acid and does not comprise NGNA.

In one embodiment the population of antibodies is substantially devoid of antibodies comprising a bisecting N-acetylglucosamine branch. In one embodiment, a population of antibodies that is substantially devoid of a distinct sugar residue comprises antibodies comprising said distinct sugar residue with a relative frequency of 2 % or less. In one embodiment, a population of antibodies that is substantially devoid of a distinct sugar residue comprises antibodies comprising said distinct sugar residue with a relative frequency of 1 % or less. In one embodiment, a population of antibodies that is substantially devoid of a distinct sugar residue comprises antibodies comprising said distinct sugar residue with a relative frequency of 0.5 % or less.

In one embodiment the population of antibodies is devoid of antibodies comprising a bisecting N-acetylglucosamine branch.

In one embodiment of the invention the population of antibodies comprises a relative frequency of 80 - 99.5 % of Fc galactosylated antibodies (including mono-galactosylated and bi-galactosylated glycan structures), a relative frequency of less than 20 % of Fc afucosylated antibodies, and optionally a relative frequency of 20 - 98 % of Fc sialylated antibodies (including mono-sialylated and bi-sialylated glycan structures). In one embodiment of the invention the population of antibodies comprises a relative frequency of 80 - 99.5 % of Fc galactosylated antibodies, a relative frequency of less than 20 % of Fc afucosylated antibodies, and optionally a relative frequency of up to 20 - 98 % of Fc sialylated antibodies, and the population of antibodies is devoid of antibodies comprising a bisecting N-acetylglucosamine branch.

In one embodiment of the invention the population of antibodies comprises a relative frequency of 80 - 99.5 % of Fc galactosylated antibodies (including mono-galactosylated and bi-galactosylated glycan structures), a relative frequency of 7 - 16 % of Fc afucosylated antibodies, optionally a relative frequency of 20 - 90 % of Fc sialylated antibodies (including mono-sialylated and bi-sialylated glycan structures). In one embodiment of the invention the population of antibodies comprises a relative frequency of 80 - 99.5 % of Fc galactosylated antibodies, a relative frequency of 7 - 16 % of Fc afucosylated antibodies, optionally a relative frequency of 20 - 90 % of Fc sialylated antibodies, and the population of antibodies is devoid of antibodies comprising a bisecting N-acetylglucosamine branch.

In one embodiment of the invention the population of antibodies comprises a relative frequency of 80 % to 95 % of Fc bi-galactosylated antibodies, a relative frequency of less than 20 % of Fc afucosylated antibodies, and optionally a relative frequency of 20 - 98 % of Fc sialylated antibodies (including mono-sialylated and bi-sialylated glycan structures). In one embodiment of the invention the population of antibodies comprises a relative frequency of 80 % to 95 % of Fc bi-galactosylated antibodies, a relative frequency of less than 20 % of Fc afucosylated antibodies, and optionally a relative frequency of 20 - 98 % of Fc sialylated antibodies (including mono-sialylated and bi-sialylated glycan structures), and the population of antibodies is devoid of antibodies comprising a bisecting N-acetylglucosamine branch.

In one embodiment the population of antibodies comprises a relative frequency of 80 % to 90 % of Fc bi-galactosylated antibodies and a relative frequency of 7 - 16 % of Fc afucosylated antibodies. In one embodiment the population of antibodies comprises a relative frequency of 80 % to 90 % of Fc bi-galactosylated antibodies and a relative frequency of 7 - 16 % of Fc afucosylated antibodies, wherein the population of antibodies is devoid of antibodies comprising a bisecting N-acetylglucosamine branch.

In one embodiment, the antibody comprises a glycosylation site at an asparagine residue located between position 294 and 300 (numbering according to EU index of Kabat) in the CH2 domains of the heavy chain. In one embodiment, the antibody comprises a glycosylation site at an asparagine residue located between position 294 and 300 (numbering according to EU index of Kabat) in the CH2 domains of the heavy chain and does not comprise further glycosylation sites.

In one embodiment of the invention the recombinant antibody does not comprise a glycosylation site in the Fab region. In one embodiment, the antibody according to the invention does not comprise a glycosylation site for N-linked glycans in the Fab region. In one embodiment, the Fab region of the antibody according to the invention does not comprise the following amino acid sequences: Asn-X-Ser and Asn-X-Thr, wherein X is an amino acid except proline, Ser is serine and Thr is threonine.

In one embodiment of the invention the antibody is of human IgG1 isotype. In one embodiment, the antibody is a human antibody. In one embodiment, the antibody is a humanized antibody. In one embodiment, the antibody is a chimeric murine/human antibody.

In one embodiment of the invention the antibody is a full length IgG1 antibody. In one embodiment, the antibody is a monoclonal antibody. In one embodiment, the antibody is a monoclonal full length antibody of human IgG1 isotype.

In one embodiment of the invention the antibody is of human IgG1 isotype with the mutations L234A and L235A (numbering according to EU index of Kabat). In one embodiment of the invention the antibody is of human IgG1 isotype with the mutations L234A, L235A and P329G (numbering according to EU index of Kabat).

In one embodiment of the invention a mode of action of the recombinant IgG1 antibody is the induction of ADCC.

In one embodiment of the invention, the antibody is a monospecific antibody.

In one embodiment of the invention, the antibody is a multispecific antibody.

In one embodiment of the invention, the antibody is a bivalent or trivalent antibody. In one embodiment of the invention, the antibody is a bivalent antibody.

In one embodiment of the invention, the antibody is selected from the group consisting of trastuzumab, rituximab, pertuzumab and obinutuzumab.

In one embodiment, the antibody specifically binds to HER2. In one embodiment, the antibody specifically binds to HER2 and includes a heavy chain variable domain of SEQ ID NO: 1 and a light chain variable domain of SEQ ID NO: 2. In one embodiment, the antibody is trastuzumab.

In one embodiment, the antibody specifically binds to CD20. In one embodiment, the antibody specifically binds to CD20 and includes a heavy chain variable domain of SEQ ID NO: 3 and a light chain variable domain of SEQ ID NO: 4. In one embodiment, the antibody is rituximab.

In one embodiment, the antibody specifically binds to HER2. In one embodiment, the antibody specifically binds to HER2 and includes a heavy chain variable domain of SEQ ID NO: 5 and a light chain variable domain of SEQ ID NO: 6. In one embodiment, the antibody is pertuzumab.

In one embodiment, the antibody specifically binds to CD20. In one embodiment, the antibody specifically binds to CD20 and includes a heavy chain variable domain of SEQ ID NO: 7 and a light chain variable domain of SEQ ID NO: 8. In one embodiment, the antibody is obinutuzumab.

In one embodiment of the invention the antibody is an *in vitro* galactoengineered antibody. In one embodiment of the invention the antibody is an *in vivo* galactoengineered antibody.

### b) Method

Another aspect of the invention is a method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) genetically engineering the host cell to improve protein galactosylation such that a population of said galactoengineered recombinant antibody is obtained, which comprises a relative frequency of at least 70 % of Fc bi-galactosylated antibodies.

Another aspect of the invention is a method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) genetically engineering the host cell to improve protein galactosylation such that a population of said galactoengineered recombinant antibody is obtained, which comprises a relative frequency of at least 80 % of Fc bi-galactosylated antibodies (including mono-galactosylated and bi-galactosylated glycan structures).

Another aspect of the invention is a method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) isolating said population of the recombinant antibody,
c) enzymatically treating said population of antibodies with galactosyltransferase to obtain a population of said galactoengineered recombinant antibody, which comprises a relative frequency of at least 80 % of Fc galactosylated antibodies (including mono-galactosylated and bi-galactosylated glycan structures) and subsequent separation of the population of said galactoengineered recombinant antibody from said enzyme.

Another aspect of the invention is a method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) isolating said population of the recombinant antibody,
c) enzymatically treating said population of antibodies with galactosyltransferase to obtain a population of said galactoengineered recombinant antibody, which comprises a relative frequency of at least 70 % of Fc bi-galactosylated antibodies and subsequent separation of the population of said galactoengineered recombinant antibodyfrom said enzyme.

In one embodiment of one of the methods according to the invention, the population of antibodies comprises a relative frequency of less than 20 % of Fc afucosylated antibodies.

Hence, in one embodiment the invention relates to a method for the production of a population of galactoengineered recombinant antibodies as described above, which comprises a relative frequency of at least 80 % of Fc galactosylated antibodies and a relative frequency of less than 20 % of Fc afucosylated antibodies.

In another embodiment the invention relates to a method for the production of a population of galactoengineered recombinant antibodies as described above, which comprises a relative frequency of at least 70 % of Fc bi-galactosylated antibodies and a relative frequency of less than 20 % of Fc afucosylated antibodies.

Above methods are suitable for generating an antibody population which comprises a high amount of galactosylated, specifically bi-galactosylated, N-linked glycan structures. By a method according to the invention, the capability of the antibody population comprising IgG1 molecules to mediate ADCC is improved. In one embodiment, the binding of said IgG1 molecules to at least one activating Fc-gammaR is improved. In one embodiment, the binding of said IgG1 molecules to Fc-gammaRIIIa is improved. In one embodiment, the binding of said IgG1 molecule to Fc-gammaRIIa is improved.

In one embodiment, said method is for the production of an antibody according to the invention.

In one embodiment of the invention, the population of galactoengineered recombinant antibodies comprises a relative frequency of less than 25 % of Fc agalactosylated antibodies. In one embodiment of the invention, the population of galactoengineered recombinant antibodies comprises up to 100 % of Fc galactosylated antibodies. In one embodiment, the population of galactoengineered recombinant antibodies comprises a relative frequency of up to 99.5 % of Fc galactosylated antibodies. In one embodiment, the population of galactoengineered recombinant antibodies comprises a relative frequency of up to 95 % of Fc galactosylated antibodies. In one embodiment, the population of galactoengineered recombinant antibodies comprises a relative frequency of up to 90 % of Fc galactosylated antibodies. In one embodiment, the population of galactoengineered recombinant antibodies comprises a relative frequency of 80 % to 99.5 % of Fc galactosylated antibodies.

In one embodiment of the invention, the population of galactoengineered recombinant antibodies comprises a relative frequency of up to 98 % of Fc bi-galactosylated antibodies. In one embodiment, the population of galactoengineered recombinant antibodies comprises a relative frequency of up to 95 % of Fc bi-galactosylated antibodies. In one embodiment, the population of galactoengineered recombinant antibodies comprises a relative frequency of up to 90 % of Fc bi-galactosylated antibodies. In one embodiment, the population of galactoengineered recombinant antibodies comprises a relative frequency of 70 % to 95 % of Fc bi-galactosylated antibodies. In one embodiment, the population of galactoengineered recombinant antibodies comprises a relative frequency of 80 % to 95 % of Fc bi-galactosylated antibodies. In one embodiment, the population of galactoengineered recombinant antibodies comprises a relative frequency of 80 % to 90 % of Fc bi-galactosylated antibodies.

In one embodiment of one of the methods according to the invention, the population of recombinant antibodies obtained in step a) comprises a relative frequency of up to 16 % of Fc afucosylated antibodies. In one embodiment of one of the methods according to the invention, the population of recombinant antibodies obtained in step a) comprises a relative frequency of at least 2 % of Fc afucosylated antibodies. In one embodiment, the population of recombinant antibodies obtained in step a) comprises a relative frequency of at least 5 % of Fc afucosylated antibodies. In one embodiment, the population of recombinant antibodies obtained in step a) comprises a relative frequency of at least 7 % of Fc afucosylated antibodies. In one embodiment, the population of recombinant antibodies obtained in step a) comprises a relative frequency of at least 8 % of Fc afucosylated antibodies. In one embodiment, the population of recombinant antibodies obtained in step a) comprises a relative frequency of less than 16 % of Fc afucosylated antibodies. In one embodiment, the population of recombinant antibodies obtained in step a) comprises a relative frequency of 2 % to 20 % of Fc afucosylated antibodies. In one embodiment, the population of recombinant antibodies obtained in step a) comprises a relative frequency of 5 % to 20 % of Fc afucosylated antibodies. In one embodiment, the population of recombinant antibodies obtained in step a) comprises a relative frequency of 7 % to 16 % of Fc afucosylated antibodies. In one embodiment, the population of recombinant antibodies obtained in step a) comprises a relative frequency of 8 % to 12 % of Fc afucosylated antibodies.

In one embodiment of the invention the recombinant antibody does not comprise a glycosylation site in the Fab region. In one embodiment, the antibody according to the invention does not comprise a glycosylation site for N-linked glycans in the Fab region. In one embodiment, the Fab region of the antibody according to the invention does not comprise the following amino acid sequences: Asn-X-Ser and Asn-X-Thr, wherein X is an amino acid except proline, Ser is serine and Thr is threonine.

In one embodiment of one of the methods according to the invention, the step regarding the genetic engineering of the host cell to improve protein galactosylation is performed by overexpressing in the host cell an enzyme capable of increasing the relative frequency of Fc bi-galactosylated antibodies. In one embodiment of one of the methods according to the invention, the enzyme capable of increasing the relative frequency of Fc bi-galactosylated antibodies is a galactosyltransferase. In one embodiment of one of the methods according to the invention, the enzyme capable of increasing the relative frequency of Fc bi-galactosylated antibodies is beta-(1-4)-Galactosyltransferase. In one embodiment of one of the methods according to the invention, the step regarding the genetic engineering of the host cell to improve protein galactosylation is performed by overexpressing in the host cell an enzyme capable of increasing the relative frequency of Fc bi-galactosylated antibodies. In one embodiment of one of the methods according to the invention, the enzyme capable of increasing the relative frequency of Fc bi-galactosylated antibodies is a galactosyltransferase. In one embodiment of one of the methods according to the invention, the enzyme capable of increasing the relative frequency of Fc bi-galactosylated antibodies is beta-(1-4)-Galactosyltransferase.

In one embodiment of one of the methods according to the invention, the step regarding the genetic engineering of the host cell to improve protein galactosylation is performed by reducing the expression or by knock-out of an enzyme in the host cell capable of decreasing the relative frequency of Fc bi-galactosylated antibodies. In one embodiment of one of the methods according to the invention, the enzyme capable of decreasing the relative frequency of Fc bi-galactosylated antibodies is a galactosidase. In one embodiment of one of the methods according to the invention, the enzyme capable of decreasing the relative frequency of Fc bi-galactosylated antibodies is β(1-4)-Galactosidase. In one embodiment of one of the methods according to the invention, the step regarding the genetic engineering of the host cell to improve protein galactosylation is performed by reducing the expression or by knock-out of an enzyme in the host cell capable of decreasing the relative frequency of Fc bi-galactosylated antibodies. In one embodiment of one of the methods according to the invention, the enzyme capable of decreasing the relative frequency of Fc bi-galactosylated antibodies is galactosidase. In one embodiment of one of the methods according to the invention, the enzyme capable of decreasing the relative frequency of Fc bi-galactosylated antibodies is β(1-4)-Galactosidase.

In one embodiment of one of the methods according to the invention, the step regarding the genetic engineering of the host cell to improve protein galactosylation is performed by overexpressing in the host cell an enzyme capable of increasing the relative frequency of Fc galactosylated antibodies. In one embodiment of one of the methods according to the invention, the enzyme capable of increasing the relative frequency of Fc galactosylated antibodies is a galactosyltransferase. In one embodiment of one of the methods according to the invention, the enzyme capable of increasing the relative frequency of Fc galactosylated antibodies is beta-(1-4)-Galactosyltransferase. In one embodiment of one of the methods according to the invention, the step regarding the genetic engineering of the host cell to improve protein galactosylation is performed by overexpressing in the host cell an enzyme capable of increasing the relative frequency of Fc galactosylated antibodies. In one embodiment of one of the methods according to the invention, the enzyme capable of increasing the relative frequency of Fc galactosylated antibodies is a galactosyltransferase. In one embodiment of one of the methods according to the invention, the enzyme capable of increasing the relative frequency of Fc galactosylated antibodies is beta-(1-4)-Galactosyltransferase.

In one embodiment of one of the methods according to the invention, the step regarding the genetic engineering of the host cell to improve protein galactosylation is performed by reducing the expression or by knock-out of an enzyme in the host cell capable of decreasing the relative frequency of Fc galactosylated antibodies. In one embodiment of one of the methods according to the invention, the enzyme capable of decreasing the relative frequency of Fc galactosylated antibodies is a galactosidase. In one embodiment of one of the methods according to the invention, the enzyme capable of decreasing the relative frequency of Fc galactosylated antibodies is β(1-4)-Galactosidase. In one embodiment of one of the methods according to the invention, the step regarding the genetic engineering of the host cell to improve protein galactosylation is performed by reducing the expression or by knock-out of an enzyme in the host cell capable of decreasing the relative frequency of Fc galactosylated antibodies. In one embodiment of one of the methods according to the invention, the enzyme capable of decreasing the relative frequency of Fc galactosylated antibodies is galactosidase. In one embodiment of one of the methods according to the invention, the enzyme capable of decreasing the relative frequency of Fc galactosylated antibodies is β(1-4)-Galactosidase.

In one embodiment of one of the methods according to the invention, genetic engineering of the host cell to improve protein galactosylation is performed by standard molecular biology techniques. In one embodiment, reducing the expression or knock-out of an enzyme in the host cell capable of decreasing the relative frequency of Fc bi-galactosylated antibodies can be performed by methods selected from the group consisting of homologous recombination, use of Zinc finger nucleases, RNA interference (RNAi) and micro RNA (miRNA). In one embodiment, increasing the relative frequency of Fc bi-galactosylated antibodies can be performed by production process development. In one embodiment, increasing the relative frequency of Fc bi-galactosylated antibodies can be performed by supplementation of galactose into the cell culture medium. In one embodiment, increasing the relative frequency of Fc bi-galactosylated antibodies can be performed by supplementation of glutamate into the cell culture medium. In one embodiment, increasing the relative frequency of Fc bi-galactosylated antibodies can be performed by substitution of glutamine by glutamate in the cell culture medium. In one embodiment, increasing the relative frequency of Fc bi-galactosylated antibodies can be performed by supplementation of manganese into the cell culture medium. In one embodiment, overexpressing an enzyme in the host cell capable of increasing the relative frequency of Fc bi-galactosylated antibodies can be performed by generating cell line stably expressing said enzyme using methotrexate selective pressure.

In one embodiment of one of the methods according to the invention, genetic engineering of the host cell to improve protein galactosylation is performed by standard molecular biology techniques. In one embodiment, reducing the expression or knock-out of an enzyme in the host cell capable of decreasing the relative frequency of Fc galactosylated antibodies can be performed by methods selected from the group consisting of homologous recombination, use of Zinc finger nucleases, RNA interference (RNAi) and micro RNA (miRNA). In one embodiment, increasing the relative frequency of Fc galactosylated antibodies can be performed by production process development. In one embodiment, increasing the relative frequency of Fc galactosylated antibodies can be performed by supplementation of galactose into the cell culture medium. In one embodiment, increasing the relative frequency of Fc galactosylated antibodies can be performed by supplementation of glutamate into the cell culture medium. In one embodiment, increasing the relative frequency of Fc galactosylated antibodies can be performed by substitution of glutamine by glutamate in the cell culture medium. In one embodiment, increasing the relative frequency of Fc galactosylated antibodies can be performed by supplementation of manganese into the cell culture medium. In one embodiment, overexpressing an enzyme in the host cell capable of increasing the relative frequency of Fc galactosylated antibodies can be performed by generating cell line stably expressing said enzyme using methotrexate selective pressure.

In one embodiment of one of the methods according to the invention, the enzymatic treatment of step c) is performed at 30 - 38 °C. In one embodiment, the enzymatic treatment of step c) is performed at 31 - 38 °C. In one embodiment, the enzymatic treatment of step c) is performed at 31 - 35 °C. In one embodiment, the enzymatic treatment of step c) is performed at about 32 °C.

In one embodiment of one of the methods according to the invention, the enzymatic treatment of step c) is performed in a solution of pH 6 - 7. In one embodiment, the enzymatic treatment of step c) is performed in a solution of pH 6.3 - 6.8. In one embodiment, the enzymatic treatment of step c) is performed a solution of about pH 6.5.

In one embodiment of one of the methods according to the invention, the enzymatic treatment of step c) is performed for at least 150 min. In one embodiment, the enzymatic treatment of step c) is performed for at least 2 h. In one embodiment, the enzymatic treatment of step c) is performed for up to 150 h. In one embodiment, the enzymatic treatment of step c) is performed at least 150 min.

In one embodiment of one of the methods according to the invention, step c) involves enzymatic treatment of said population of antibodies with galactosyltransferase in a solution of pH 6 - 7, at 30 - 35 °C for at least 150 min. In one embodiment of one of the methods according to the invention, step c) involves enzymatic treatment of said population of antibodies with galactosyltransferase in a solution of pH 6.3 - 6.8, at 31 - 34 °C for at least 150 min. In one embodiment of one of the methods according to the invention, step c) involves enzymatic treatment of said population of antibodies with galactosyltransferase in a solution of pH 6.3 - 6.8, at 31 - 34 °C for 2 - 150 h. In one embodiment of one of the methods according to the invention, step c) involves enzymatic treatment of said population of antibodies with galactosyltransferase in a solution of about pH 6.5, at about 32 °C for 2 - 150 h.

In one embodiment, step c) is performed in the presence of galactosyltransferase and UDP-galactose (Uridine diphosphate galactose). In one embodiment, step c) is performed in the presence of galactosyltransferase, UDP-galactose and MnCl₂.

In one embodiment the galactosyltransferase is a beta(1-4)-galactosyltransferase capable of transferring galactose from UDP-galactose to N-acetylglucosamine residues of oligosaccharides.

In one embodiment of one of the methods according to the invention, the antibodies of IgG1 isotype are recombinantly produced in a host cell, wherein the host cell is not genetically engineered to improve or impair protein fucosylation. In one embodiment, the antibodies are produced in host cells, wherein the host cells do not exhibit altered expression (e.g. depletion) of fucose transferase resulting from a genetic alteration of the host cell, and wherein the host cells are capable of expression at least a fraction of fucosylated proteins.

In one embodiment of one of the methods according to the invention, the host cell is a eukaryotic cell. In one embodiment, the host cell is a CHO cell or a mouse myeloma cell. In one embodiment, the host cell is a CHO cell. In one embodiment, the host cell is a CHO cell that is not genetically engineered to improve or impair protein fucosylation. In one embodiment, the host cell is a CHO cell that does not exhibit altered expression (e.g. depletion) of fucose transferase resulting from a genetic alteration of the CHO cell, and wherein the CHO cell is capable of expression at least a fraction of fucosylated proteins.

In one embodiment, the host cell is capable of expressing a population of antibodies with a relative frequency of up to 20 % of Fc afucosylated antibodies. In one embodiment, the host cell is capable of expressing a population of antibodies with a relative frequency of up to 16 % of Fc afucosylated antibodies. In one embodiment the host cell is capable of expressing a population of antibodies with a relative frequency of at least 2 % of Fc afucosylated antibodies. In one embodiment, the host cell is capable of expressing a population of antibodies with a relative frequency of at least 5 % of Fc afucosylated antibodies. In one embodiment, the host cell is capable of expressing a population of antibodies with a relative frequency of at least 7 % of Fc afucosylated antibodies. In one embodiment, the host cell is capable of expressing a population of antibodies with a relative frequency of at least 8 % of Fc afucosylated antibodies. In one embodiment, the host cell is capable of expressing a population of antibodies with a relative frequency of 5 % to 20 % of Fc afucosylated antibodies. In one embodiment, the host cell is capable of expressing a population of antibodies with a relative frequency of 7 % to 16 % of Fc afucosylated antibodies. In one embodiment, the host cell is capable of expressing a population of antibodies with a relative frequency of 8 % to 12 % of Fc afucosylated antibodies.

In one embodiment, a method according to the invention does not include the *in vitro* addition or *in vitro* cleavage of a fucose residue from the from the N-linked glycan structure of antibodies. In one embodiment, the host cell is not genetically engineered to improve or impair protein fucosylation and the method according to the invention does not include the *in vitro* addition or *in vitro* cleavage of a fucose residue from the from the N-linked glycan structure of antibodies. In one embodiment, the host cell is a CHO cell that is not genetically engineered to improve or impair protein fucosylation and the method according to the invention does not include the *in vitro* addition or *in vitro* cleavage of a fucose residue from the from the N-linked glycan structure of antibodies.

Hence, in one embodiment of one of the methods according to the invention the population of galactoengineered recombinant antibodies comprises a relative frequency of 80 - 99.5 % of Fc galactosylated antibodies (including mono-galactosylated and bi-galactosylated glycan structures) and a relative frequency of less than 20 % of Fc afucosylated fucosylated antibodies. In one embodiment the population of galactoengineered recombinant antibodies comprises a relative frequency of 80 - 99.5 % of Fc galactosylated antibodies and a relative frequency less than 20 % of Fc afucosylated antibodies, and the population of antibodies is devoid of antibodies comprising a bisecting N-acetylglucosamine branch.

In one embodiment of the invention the population of galactoengineered recombinant antibodies comprises a relative frequency of 80 - 99.5 % of Fc galactosylated antibodies and a relative frequency of 7 - 16 % of Fc afucosylated antibodies. In one embodiment the population of galactoengineered recombinant antibodies comprises a relative frequency of 80 - 99.5 % of Fc galactosylated antibodies, a relative frequency of 7 - 16 % of Fc afucosylated antibodies, and the population of antibodies is devoid of antibodies comprising a bisecting N-acetylglucosamine branch.

In one embodiment of the invention the population of galactoengineered recombinant antibodies comprises a relative frequency of 80 % to 95 % of Fc bi-galactosylated antibodies and a relative frequency of less than 20 % of Fc afucosylated antibodies. In one embodiment of the invention the population of galactoengineered recombinant antibodies comprises a relative frequency of up to 80 % to 95 % of Fc bi-galactosylated antibodies, a relative frequency of less than 20 % of Fc afucosylated antibodies, and the population of antibodies is devoid of antibodies comprising a bisecting N-acetylglucosamine branch.

In one embodiment the population of galactoengineered recombinant antibodies comprises a relative frequency of 80 % to 90 % of Fc bi-galactosylated antibodies and a relative frequency of 7 - 16 % of Fc afucosylated antibodies. In one embodiment the population of galactoengineered recombinant antibodies comprises a relative frequency of 80 % to 90 % of Fc bi-galactosylated antibodies, a relative frequency of 7 - 16 % of Fc afucosylated antibodies, and the population of antibodies is devoid of antibodies comprising a bisecting N-acetylglucosamine branch.

In one embodiment of a method according to the invention, the antibodies are recombinantly produced in CHO cells and the population of galactoengineered recombinant antibodies comprises a relative frequency of 80 - 99.5 % of Fc galactosylated antibodies (including mono-galactosylated and bi-galactosylated glycan structures) and a relative frequency of less than 20 % of Fc afucosylated fucosylated antibodies. In one embodiment of a method according to the invention, the antibodies are recombinantly produced in CHO cells and the population of galactoengineered recombinant antibodies comprises a relative frequency of 80 - 99.5 % of Fc galactosylated antibodies and a relative frequency less than 20 % of Fc afucosylated antibodies, and the population of antibodies is devoid of antibodies comprising a bisecting N-acetylglucosamine branch.

In one embodiment of a method according to the invention, the antibodies are recombinantly produced in CHO cells and the population of galactoengineered recombinant antibodies comprises a relative frequency of 80 % to 95 % of Fc bi-galactosylated antibodies and a relative frequency of less than 20 % of Fc afucosylated antibodies. In one embodiment of a method according to the invention, the antibodies are recombinantly produced in CHO cells and the population of galactoengineered recombinant antibodies comprises a relative frequency of up to 80 % to 95 % of Fc bi-galactosylated antibodies, a relative frequency of less than 20 % of Fc afucosylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6 - 7, at 30 - 38 °C for at least 150 min, whereby the antibody population obtained in step c) comprises a relative frequency of 80 - 99.5 % of Fc galactosylated antibodies and a relative frequency less than 20 % of Fc afucosylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6 - 7, at 30 - 38 °C for at least 150 min, whereby the antibody population obtained in step c) comprises a relative frequency of up to 80 % to 95 % of Fc bi-galactosylated antibodies, a relative frequency of less than 20 % of Fc afucosylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6.3 - 6.8, at 31 - 35 °C for at least 150 min, whereby the antibody population obtained in step c) comprises a relative frequency of 80 - 99.5 % of Fc galactosylated antibodies and a relative frequency less than 20 % of Fc afucosylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6.3 - 6.8, at 31 - 35 °C for at least 150 min, whereby the antibody population obtained in step c) comprises a relative frequency of up to 80 % to 95 % of Fc bi-galactosylated antibodies, a relative frequency of less than 20 % of Fc afucosylated antibodies, and the population of antibodies is devoid of antibodies comprising a bisecting N-acetylglucosamine branch.

In one embodiment of one of the methods according to the invention the population of antibodies obtained in step c) (the N-linked glycan structures of which are hypergalactosylated) are subjected to step d) an enzymatic treatment with sialyltransferase to obtain an antibody population, which comprises a relative frequency of up to 98 % of Fc sialylated antibodies (including mono-sialylated and bi-sialylated glycan structures) and subsequent separation of the population of sialylated antibodies from said sialyltransferase.

This step d) is advantageous for generation of a population of antibodies including a high relative frequency of sialylated IgG1 molecules. By using CMP-NANA (cytidine-5'-monophospho-N-acetylneuraminic acid) as activated sugar, a homogeneous population including only NANA residues can be generated advantageously.

In one embodiment, the enzymatic treatment with sialyltransferase is performed at 35 - 40 °C. In one embodiment, the treatment with sialyltransferase is performed at 36 - 38 °C. In one embodiment, the treatment with sialyltransferase is performed at about 37 °C.

In one embodiment, the treatment with sialyltransferase is performed in a solution of pH 6 - 9. In one embodiment, the treatment with sialyltransferase is performed in a solution of pH 6.5 - 8.5. In one embodiment, the treatment with sialyltransferase is performed a solution of about pH 7 to about pH 8.

In one embodiment, the treatment with sialyltransferase is performed for at least 150 min. In one embodiment, the treatment with sialyltransferase is performed for at least 2 h. In one embodiment, the treatment with sialyltransferase is performed for 2 - 120 h.

In one embodiment, the enzymatic treatment with sialyltransferase is performed at the following conditions:
i) in a first step, sialyltransferase and the population of antibodies obtained in step c) of one of the methods according to the invention are contacted in solution at above indicated temperatures for least 150 min (in one embodiment 2 - 80 h) at pH 6 - 8 (in one embodiment pH 6.5 - 7.5); and
ii) optionally in a second step, sialyltransferase and the population of antibodies obtained in step i) are contacted in solution at above indicated temperatures for least 150 min (in one embodiment for 2 - 10 h).

By treatment with sialyltransferase including steps i) an antibody population with a high relative frequency of mono-sialylated N-linked glycan structures can be obtained. Hence, in one embodiment the treatment with sialyltransferase includes (consists of) step i). Further treatment including step ii) as indicated above results in the generation of an antibody population with a high relative frequency of bi-sialylated N-linked glycan structures. Hence, in one embodiment the treatment with sialyltransferase includes steps i) and ii).

In one embodiment, the treatment with sialyltransferase indicated in steps i) or, optionally, i) and ii) is performed for a total time of up to 120 h.

In one embodiment, step d) is performed in the presence of sialyltransferase and CMP-NANA.

In one embodiment the sialyltransferase is an alpha(2-6)-sialyltransferase, capable of transferring sialic acid (NANA) from CMP-NANA to terminal galactose residues of oligosaccharides. In one embodiment the sialyltransferase is an alpha(2-3)-sialyltransferase, capable of transferring sialic acid (NANA) from CMP-NANA to terminal galactose residues of oligosaccharides.

In one embodiment, step i) is performed using alpha(2-6)-sialyltransferase comprising an amino acid sequence according to SEQ ID NO: 10. In one embodiment, step ii) is performed using alpha(2-6)-sialyltransferase comprising an amino acid sequence according to SEQ ID NO: 9. In one embodiment, the method comprises steps i) and ii) and step i) is performed using alpha(2-6)-sialyltransferase comprising an amino acid sequence according to SEQ ID NO: 10, and step ii) is performed using alpha(2-6)-sialyltransferase comprising an amino acid sequence according to SEQ ID NO: 9.

In one embodiment of the invention the population of antibodies obtained in step d) comprises a relative frequency of up to 97 % of Fc sialylated antibodies. In one embodiment, the population of antibodies obtained in step d) comprises a relative frequency of up to 95 % of Fc sialylated antibodies. In one embodiment, the population of antibodies obtained in step d) comprises a relative frequency of up to 90 % of Fc sialylated antibodies. In one embodiment, the population of antibodies obtained in step d) comprises a relative frequency of 50 % to 90 % of Fc sialylated antibodies. In one embodiment, the population of antibodies obtained in step d) comprises a relative frequency of 50 % to 80 % of Fc sialylated antibodies.

In one embodiment, the sialic acid is 5-N-acetylneuraminic acid (NANA) or 5-N-glycolylneuraminic acid (NGNA). In one embodiment, the sialic acid is NANA. In one embodiment, the sialic acid is NGNA.

In one embodiment of a method according to the invention, the antibody population obtained in step d) comprises a relative frequency of 80 - 99.5 % of Fc galactosylated antibodies, a relative frequency of less than 20 % of Fc afucosylated antibodies, and a relative frequency of 70 % to 90 % of Fc sialylated antibodies. In one embodiment of a method according to the invention, the antibodies are recombinantly produced in CHO cells and the antibody population obtained in step d) comprises a relative frequency of 80 - 99.5 % of Fc galactosylated antibodies, a relative frequency of less than 20 % of Fc afucosylated antibodies, and a relative frequency of 50 % to 90 % of Fc sialylated antibodies, wherein the population of antibodies is devoid of antibodies comprising a bisecting N-acetylglucosamine branch.

In one embodiment of a method according to the invention, the antibody population obtained in step d) comprises a relative frequency of 80 - 99.5 % of Fc galactosylated antibodies, a relative frequency of 7 - 16 % of Fc afucosylated antibodies, and a relative frequency of 50 % to 90 % of Fc sialylated antibodies. In one embodiment of a method according to the invention, the antibodies are recombinantly produced in CHO cells and the antibody population obtained in step d) comprises a relative frequency of 80 - 99.5 % of Fc galactosylated antibodies, a relative frequency of 7 - 16 % of Fc afucosylated antibodies, and a relative frequency of 50 % to 90 % of Fc sialylated antibodies, wherein the population of antibodies is devoid of antibodies comprising a bisecting N-acetylglucosamine branch.

In one embodiment of a method according to the invention, the antibody population obtained in step d) comprises a relative frequency of 80 % to 95 % of Fc bi-galactosylated antibodies, and a relative frequency of less than 20 % of Fc afucosylated antibodies, and a relative frequency of 50 % to 90 % of Fc sialylated antibodies. In one embodiment of a method according to the invention, the antibody population obtained in step d) comprises a relative frequency of 80 % to 95 % of Fc bi-galactosylated antibodies, a relative frequency of less than 20 % of Fc afucosylated antibodies, and a relative frequency of 50 % to 90 % of Fc sialylated antibodies, wherein the population of antibodies is devoid of antibodies comprising a bisecting N-acetylglucosamine branch.

In one embodiment of a method according to the invention, the antibody population obtained in step d) comprises a relative frequency of 80 % to 90 % of Fc bi-galactosylated antibodies, and a relative frequency of 7 - 16 % of Fc afucosylated antibodies, and a relative frequency of 50 % to 90 % of Fc sialylated antibodies. In one embodiment of a method according to the invention, the antibody population obtained in step d) comprises a relative frequency of 80 % to 90 % of Fc bi-galactosylated antibodies, a relative frequency of 7 - 16 % of Fc afucosylated antibodies, and a relative frequency of 50 % to 90 % of Fc sialylated antibodies, wherein the population of antibodies is devoid of antibodies comprising a bisecting N-acetylglucosamine branch.

In one embodiment of a method according to the invention, the antibodies are recombinantly produced in CHO cells and the population of galactoengineered recombinant antibodies comprises a relative frequency of 80 - 99.5 % of Fc galactosylated antibodies (including mono-galactosylated and bi-galactosylated glycan structures), a relative frequency of less than 20 % of Fc afucosylated fucosylated antibodies and a relative frequency of 50 % to 90 % of Fc sialylated antibodies. In one embodiment of a method according to the invention, the antibodies are recombinantly produced in CHO cells and the population of galactoengineered recombinant antibodies comprises a relative frequency of 80 - 99.5 % of Fc galactosylated antibodies, a relative frequency less than 20 % of Fc afucosylated antibodies, and a relative frequency of 50 % to 90 % of Fc sialylated antibodies, and the population of antibodies is devoid of antibodies comprising a bisecting N-acetylglucosamine branch.

In one embodiment of a method according to the invention, the antibodies are recombinantly produced in CHO cells and the population of galactoengineered recombinant antibodies comprises a relative frequency of 80 % to 95 % of Fc bi-galactosylated antibodies, a relative frequency of less than 20 % of Fc afucosylated antibodies and a relative frequency of 50 % to 90 % of Fc sialylated antibodies. In one embodiment of a method according to the invention, the antibodies are recombinantly produced in CHO cells and the population of galactoengineered recombinant antibodies comprises a relative frequency of 80 % to 95 % of Fc bi-galactosylated antibodies, a relative frequency of less than 20 % of Fc afucosylated antibodies, a relative frequency of 50 % to 90 % of Fc sialylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6 - 7, at 30 - 38 °C for at least 150 min, whereby the antibody population obtained in step c) comprises a relative frequency of 80 - 99.5 % of Fc galactosylated antibodies, a relative frequency less than 20 % of Fc afucosylated antibodies, a relative frequency of 50 % to 90 % of Fc sialylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6 - 7, at 30 - 38 °C for at least 150 min, whereby the antibody population obtained in step c) comprises a relative frequency of up to 80 % to 95 % of Fc bi-galactosylated antibodies, a relative frequency of less than 20 % of Fc afucosylated antibodies, a relative frequency of 50 % to 90 % of Fc sialylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6.3 - 6.8, at 31 - 35 °C for at least 150 min, whereby the antibody population obtained in step c) comprises a relative frequency of 80 - 99.5 % of Fc galactosylated antibodies, a relative frequency less than 20 % of Fc afucosylated antibodies, a relative frequency of 50 % to 90 % of Fc sialylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6.3 - 6.8, at 31 - 35 °C for at least 150 min, whereby the antibody population obtained in step c) comprises a relative frequency of up to 80 % to 95 % of Fc bi-galactosylated antibodies, a relative frequency of less than 20 % of Fc afucosylated antibodies, and a relative frequency of 50 % to 90 % of Fc sialylated antibodies, and the population of antibodies is devoid of antibodies comprising a bisecting N-acetylglucosamine branch.

In one embodiment of a method according to the invention, the antibodies are recombinantly produced in CHO cells and step c) involves enzymatic treatment of said population of antibodies with galactosyltransferase in a solution of pH 6 - 7, at 30 - 38 °C for at least 150 min, and the enzymatic treatment with sialyltransferase is performed at the following conditions:
i) sialyltransferase and the population of antibodies obtained in step c) are contacted in solution at above indicated temperatures for at least 150 min (in one embodiment for 2 - 120 h) at pH 6 - 8 (in one embodiment pH 6.5 - 7.5).

In one embodiment of a method according to the invention, the antibodies are recombinantly produced in CHO cells and step c) involves enzymatic treatment of said population of antibodies with galactosyltransferase in a solution of pH 6.3 - 6.8, at 31 - 35 °C for at least 150 min, and the enzymatic treatment with sialyltransferase is performed at the following conditions:
i) in a first step, sialyltransferase and the population of antibodies obtained in step c) are contacted in solution at above indicated temperatures for at least 150 min (in one embodiment for 2 - 120 h) at pH 6 - 8 (in one embodiment pH 6.5 - 7.5); and
ii) in a second step, sialyltransferase and the population of antibodies obtained in step i) are contacted in solution at above indicated temperatures for further at least 150 min (in one embodiment for 2 - 10 h) at pH 6 - 8 (in one embodiment pH 6.5 - 7.5).

In one embodiment, a method according to the invention encompasses addition of at least one galactose residue to at least a fraction of the antibodies comprised within the population of antibodies that was subject of galactoengineering. In one embodiment, a method according to the invention encompasses addition of at least one galactose residue to at least a fraction of the antibodies comprised within the population of antibodies that was subject of galactoengineeringso as to achieve a relative frequency of at least 80 % of Fc galactosylated antibodies. In one embodiment, a method according to the invention encompasses addition of at least one galactose residue to at least a fraction of the antibodies comprised within the population of antibodies that was subject of galactoengineeringso as to achieve a relative frequency of at least 70 % of Fc bi-galactosylated antibodies.

In one embodiment of a method according to the invention, the antibody is selected from the group consisting of trastuzumab, rituximab, pertuzumab and obinutuzumab.Another aspect of the invention is the population of galactoengineered recombinant antibodies of IgG1 isotype obtainable by a method according the invention.

### c) Pharmaceutical composition, treatment

Another aspect of the invention is a pharmaceutical composition comprising a population of galactoengineeredrecombinant antibodies of IgG1 isotype according to the invention. One aspect of the invention is a pharmaceutical composition a population of galactoengineeredrecombinant antibodies of IgG1 isotype according to the invention in combination with at least one pharmaceutically acceptable carrier.

Another aspect of the invention is the pharmaceutical composition according to the invention for use as a medicament. Another aspect of the invention is the pharmaceutical composition according to the invention for use in the treatment of cancer. Another aspect of the invention is the pharmaceutical composition according to the invention for use in the treatment of an inflammatory disorder. Another aspect of the invention is the pharmaceutical composition according to the invention for use in the treatment of an autoimmune disorder.

Another aspect of the invention is a method of treatment of a patient suffering from a disease by administering a population of galactoengineeredrecombinant antibodies of IgG1 isotype according to the invention to the patient in the need of such treatment. Another aspect of the invention is a method of treatment of a patient suffering from cancer disease by administering a population of galactoengineeredrecombinant antibodies of IgG1 isotype according to the invention to the patient in the need of such treatment. Another aspect of the invention is a method of treatment of a patient suffering from an inflammatory disorder by administering a population of galactoengineered recombinant antibodies of IgG1 isotype according to the invention to the patient in the need of such treatment. Another aspect of the invention is a method of treatment of a patient suffering from an autoimmune disorder by administering a population of galactoengineered recombinant antibodies of IgG1 isotype according to the invention to the patient in the need of such treatment.

Another aspect of the invention is a method of treatment of a patient suffering from a disease by administering a pharmaceutical composition according to the invention to the patient in the need of such treatment. Another aspect of the invention is a method of treatment of a patient suffering from cancer by administering a pharmaceutical composition according to the invention to the patient in the need of such treatment. Another aspect of the invention is a method of treatment of a patient suffering from an inflammatory disorder by administering a pharmaceutical composition according to the invention to the patient in the need of such treatment. Another aspect of the invention is a method of treatment of a patient suffering from an autoimmune disorder by administering a pharmaceutical composition according to the invention to the patient in the need of such treatment.

### d) Uses of antibodies and methods of the invention

Another aspect of the invention is the use of the method according to the invention for improving ADCC mediated by said population of recombinant antibodies of IgG1 isotype.

Another aspect of the invention is the use of the method according to the invention for improving the binding affinity of recombinant antibodies to Fc-gammaRIIa and Fc-gammaRIIIa.

Another aspect of the invention is the use of a population of galactoengineered recombinant antibodies according to the invention for mediating ADCC. Another aspect of the invention is the use of a population of galactoengineered recombinant antibodies according to the invention for recruiting and / or activating natural killer cells. Another aspect of the invention is the use of a population of galactoengineered recombinant antibodies according to the invention for activation of Fc-gammaR-comprising effector cells.

Another aspect of the invention is the population of galactoengineered recombinant antibodies according to the invention for use as a medicament. Another aspect of the invention is the population of galactoengineered recombinant antibodies according to the invention for use in the treatment of cancer. Another aspect of the invention is the population of galactoengineered recombinant antibodies according to the invention for use in the treatment of an inflammatory disorder. Another aspect of the invention is the population of galactoengineered recombinant antibodies according to the invention for use in the treatment of an autoimmune disorder.

### 3. Specific embodiments of the invention

The following items further provide specific aspects of the disclosure, and specific embodiments to practice the teachings provided herein.
1. A population of galactoengineered recombinant antibodies of IgG1 isotype, comprising a relative frequency of at least 70 % of Fc bi-galactosylated antibodies and a relative frequency of less than 20 % of Fc afucosylated antibodies.
2. The population of antibodies according to embodiment 1, comprising a relative frequency of 80 - 95 % of Fc bi-galactosylated antibodies and a relative frequency of less than 20 % of Fc afucosylated antibodies.
3. The population of antibodies according to embodiment 1, comprising a relative frequency of 80 - 95 % of Fc bi-galactosylated antibodies and a relative frequency of 7 - 16 % of Fc afucosylated antibodies.
4. The population of antibodies according to embodiment 1, comprising a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
5. The population of antibodies according to embodiment 1, comprising a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
6. The population of antibodies according to any one of embodiments 1 to 5, wherein the population of antibodies is devoid of antibodies comprising a bisecting N-acetylglucosamine branch.
7. The population of antibodies according to any one of embodiments 1 to 6, wherein the Fab region of the antibody does not comprise the following amino acid sequences: Asn-X-Ser and Asn-X-Thr, wherein X is an amino acid except proline, Ser is serine and Thr is threonine.
8. The population of antibodies according to any one of embodiments 1 to 7, wherein the antibody is a monoclonal full length antibody of human IgG1 isotype.
9. The population of antibodies according to any one of embodiments 1 to 7, wherein the antibody is a humanized antibody.
10. The population of antibodies according to any one of embodiments 1 to 7, wherein the antibody is selected from the group consisting of trastuzumab, rituximab, pertuzumab and obinutuzumab.
11. The population of antibodies according to any one of embodiments 1 to 10, wherein the antibody is a *in vitro* galactoengineered recombinant antibody.
12. The population of antibodies according to any one of embodiments 1 to 10, wherein the antibody is a *in vivo* galactoengineered recombinant antibody.
13. A population of galactoengineered recombinant antibodies of IgG1 isotype, comprising a relative frequency of at least 80 % of Fc galactosylated antibodies and a relative frequency of less than 20 % of Fc afucosylated antibodies.
14. The population of antibodies according to embodiment 13, comprising a relative frequency of 80 - 99.5 % of Fc galactosylated antibodies and a relative frequency of less than 20 % of Fc afucosylated antibodies.
15. The population of antibodies according to embodiment 13, comprising a relative frequency of 80 - 99.5 % of Fc galactosylated antibodies and a relative frequency of 7 - 16 % of Fc afucosylated antibodies.
16. The population of antibodies according to embodiment 13, comprising a relative frequency of 80 - 99.5 % of Fc galactosylated antibodies, a relative frequency of less than 20 % of Fc afucosylated antibodies, and a relative frequency of 50 % to 90 % of Fc sialylated antibodies.
17. The population of antibodies according to embodiment 13, comprising a relative frequency of 80 - 99.5 % of Fc galactosylated antibodies, a relative frequency of 7 - 16 % of Fc afucosylated antibodies, and a relative frequency of 50 % to 90 % of Fc sialylated antibodies.
18. The population of antibodies according to any one of embodiments 13 to 17, wherein the population of antibodies is devoid of antibodies comprising a bisecting N-acetylglucosamine branch.
19. The population of antibodies according to any one of embodiments 13 to 18, wherein the Fab region of the antibody does not comprise the following amino acid sequences: Asn-X-Ser and Asn-X-Thr, wherein X is an amino acid except proline, Ser is serine and Thr is threonine.
20. The population of antibodies according to any one of embodiments 13 to 19, wherein the antibody is a monoclonal full length antibody of human IgG1 isotype.
21. The population of antibodies according to any one of embodiments 13 to 20, wherein the antibody is a humanized antibody.
22. The population of antibodies according to any one of embodiments 13 to 21, wherein the antibody is selected from the group consisting of trastuzumab, rituximab, pertuzumab and obinutuzumab.
23. The population of antibodies according to any one of embodiments 13 to 21, wherein the antibody is an *in vitro* galactoengineered recombinant antibody.
24. The population of antibodies according to any one of embodiments 13 to 21, wherein the antibody is a *in vivo* galactoengineered recombinant antibody.
25. A population of galactoengineered recombinant antibodies of IgG1 isotype, wherein the antibody specifically binds to HER2 and comprises a heavy chain variable domain of SEQ ID NO: 1 and a light chain variable domain of SEQ ID NO: 2, comprising a relative frequency of at least 70 % of Fc bi-galactosylated antibodies and a relative frequency of less than 20 % of Fc afucosylated antibodies.
26. A population of galactoengineered recombinant antibodies of IgG1 isotype, wherein the antibody specifically binds to CD20 and comprises a heavy chain variable domain of SEQ ID NO: 3 and a light chain variable domain of SEQ ID NO: 4, comprising a relative frequency of at least 70 % of Fc bi-galactosylated antibodies and a relative frequency of less than 20 % of Fc afucosylated antibodies.
27. A population of galactoengineered recombinant antibodies of IgG1 isotype, wherein the antibody specifically binds to HER2 and comprises a heavy chain variable domain of SEQ ID NO: 5 and a light chain variable domain of SEQ ID NO: 6, comprising a relative frequency of at least 70 % of Fc bi-galactosylated antibodies and a relative frequency of less than 20 % of Fc afucosylated antibodies.
28. A population of galactoengineered recombinant antibodies of IgG1 isotype, wherein the antibody specifically binds to CD20 and comprises a heavy chain variable domain of SEQ ID NO: 7 and a light chain variable domain of SEQ ID NO: 8, comprising a relative frequency of at least 70 % of Fc bi-galactosylated antibodies and a relative frequency of less than 20 % of Fc afucosylated antibodies.
29. The population of antibodies according to any one of embodiments 25 to 28, comprising a relative frequency of 80 - 95 % of Fc bi-galactosylated antibodies and a relative frequency of less than 20 % of Fc afucosylated antibodies.
30. The population of antibodies according to any one of embodiments 25 to 28, comprising a relative frequency of 80 - 95 % of Fc bi-galactosylated antibodies and a relative frequency of 7 - 16 % of Fc afucosylated antibodies.
31. The population of antibodies according to any one of embodiments 25 to 28, comprising a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
32. The population of antibodies according to any one of embodiments 25 to 28, comprising a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
33. The population of antibodies according to any one of embodiments 25 to 32, wherein the population of antibodies is devoid of antibodies comprising a bisecting N-acetylglucosamine branch.
34. The population of antibodies according to any one of embodiments 25 to 33, wherein the Fab region of the antibody does not comprise the following amino acid sequences: Asn-X-Ser and Asn-X-Thr, wherein X is an amino acid except proline, Ser is serine and Thr is threonine.
35. The population of antibodies according to any one of embodiments 25 to 34, wherein the antibody is a monoclonal full length antibody of human IgG1 isotype.
36. The population of antibodies according to any one of embodiments 25 to 35, wherein the antibody is a humanized antibody.
37. The population of antibodies according to any one of embodiments 25 to 36, wherein the antibody is an *in vitro* galactoengineered recombinant antibody.
38. The population of antibodies according to any one of embodiments 25 to 36, wherein the antibody is an *in vivo* galactoengineered recombinant antibody.
39. A population of galactoengineered recombinant antibodies of IgG1 isotype, wherein the antibody specifically binds to HER2 and comprises a heavy chain variable domain of SEQ ID NO: 1 and a light chain variable domain of SEQ ID NO: 2, comprising a relative frequency of at least 80 % of Fc galactosylated antibodies and a relative frequency of less than 20 % of Fc afucosylated antibodies.
40. A population of galactoengineered recombinant antibodies of IgG1 isotype, wherein the antibody specifically binds to CD20 and comprises a heavy chain variable domain of SEQ ID NO: 3 and a light chain variable domain of SEQ ID NO: 4, comprising a relative frequency of at least 80 % of Fc galactosylated antibodies and a relative frequency of less than 20 % of Fc afucosylated antibodies.
41. A population of galactoengineered recombinant antibodies of IgG1 isotype, wherein the antibody specifically binds to HER2 and comprises a heavy chain variable domain of SEQ ID NO: 5 and a light chain variable domain of SEQ ID NO: 6, comprising a relative frequency of at least 80 % of Fc galactosylated antibodies and a relative frequency of less than 20 % of Fc afucosylated antibodies.
42. A population of galactoengineered recombinant antibodies of IgG1 isotype, wherein the antibody specifically binds to CD20 and comprises a heavy chain variable domain of SEQ ID NO: 7 and a light chain variable domain of SEQ ID NO: 8, comprising a relative frequency of at least 80 % of Fc galactosylated antibodies and a relative frequency of less than 20 % of Fc afucosylated antibodies.
43. The population of antibodies according to any one of embodiments 39 to 42, comprising a relative frequency of 80 - 99.5 % of Fc galactosylated antibodies and a relative frequency of less than 20 % of Fc afucosylated antibodies.
44. The population of antibodies according to any one of embodiments 39 to 42, comprising a relative frequency of 80 - 99.5 % of Fc galactosylated antibodies and a relative frequency of 7 - 16 % of Fc afucosylated antibodies.
45. The population of antibodies according to any one of embodiments 39 to 42, comprising a relative frequency of 80 - 99.5 % of Fc galactosylated antibodies, a relative frequency of less than 20 % of Fc afucosylated antibodies, and a relative frequency of 50 % to 90 % of Fc sialylated antibodies.
46. The population of antibodies according to any one of embodiments 39 to 42, comprising a relative frequency of 80 - 99.5 % of Fc galactosylated antibodies, a relative frequency of 7 - 16 % of Fc afucosylated antibodies, and a relative frequency of 50 % to 90 % of Fc sialylated antibodies.
47. The population of antibodies according to any one of embodiments 39 to 46, wherein the population of antibodies is devoid of antibodies comprising a bisecting N-acetylglucosamine branch.
48. The population of antibodies according to any one of embodiments 39 to 47, wherein the Fab region of the antibody does not comprise the following amino acid sequences: Asn-X-Ser and Asn-X-Thr, wherein X is an amino acid except proline, Ser is serine and Thr is threonine.
49. The population of antibodies according to any one of embodiments 39 to 48, wherein the antibody is a monoclonal full length antibody of human IgG1 isotype.
50. The population of antibodies according to any one of embodiments 39 to 49, wherein the antibody is a humanized antibody.
51. The population of antibodies according to any one of embodiments 39 to 50, wherein the antibody is an *in vitro* galactoengineered recombinant antibody.
52. The population of antibodies according to any one of embodiments 39 to 50, wherein the antibody is an *in vivo* galactoengineered recombinant antibody.
53. A population of galactoengineered trastuzumab, comprising a relative frequency of at least 70 % of Fc bi-galactosylated antibodies and a relative frequency of less than 20 % of Fc afucosylated antibodies.
54. A population of galactoengineered rituximab, comprising a relative frequency of at least 70 % of Fc bi-galactosylated antibodies and a relative frequency of less than 20 % of Fc afucosylated antibodies.
55. A population of galactoengineered pertuzumab, comprising a relative frequency of at least 70 % of Fc bi-galactosylated antibodies and a relative frequency of less than 20 % of Fc afucosylated antibodies.
56. A population of galactoengineered obinutuzumab, comprising a relative frequency of at least 70 % of Fc bi-galactosylated antibodies and a relative frequency of less than 20 % of Fc afucosylated antibodies.
57. The population of antibodies according to any one of embodiments 53 to 56, comprising a relative frequency of 80 - 95 % of Fc bi-galactosylated antibodies and a relative frequency of less than 20 % of Fc afucosylated antibodies.
58. The population of antibodies according to any one of embodiments 53 to 56, comprising a relative frequency of 80 - 95 % of Fc bi-galactosylated antibodies and a relative frequency of 7 - 16 % of Fc afucosylated antibodies.
59. The population of antibodies according to any one of embodiments 53 to 56, comprising a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
60. The population of antibodies according to any one of embodiments 53 to 56, comprising a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
61. The population of antibodies according to any one of embodiments 53 to 60, wherein the population of antibodies is devoid of antibodies comprising a bisecting N-acetylglucosamine branch.
62. A population of galactoengineered trastuzumab, comprising a relative frequency of at least 80 % of Fc galactosylated antibodies and a relative frequency of less than 20 % of Fc afucosylated antibodies.
63. A population of galactoengineered rituximab, comprising a relative frequency of at least 80 % of Fc galactosylated antibodies and a relative frequency of less than 20 % of Fc afucosylated antibodies.
64. A population of galactoengineered pertuzumab, comprising a relative frequency of at least 80 % of Fc galactosylated antibodies and a relative frequency of less than 20 % of Fc afucosylated antibodies.
65. A population of galactoengineered obinutuzumab, comprising a relative frequency of at least 80 % of Fc galactosylated antibodies and a relative frequency of less than 20 % of Fc afucosylated antibodies.
66. The population of antibodies according to any one of embodiments 62 to 65, comprising a relative frequency of 80 - 99.5 % of Fc galactosylated antibodies and a relative frequency of less than 20 % of Fc afucosylated antibodies.
67. The population of antibodies according to any one of embodiments 62 to 65, comprising a relative frequency of 80 - 99.5 % of Fc galactosylated antibodies and a relative frequency of 7 - 16 % of Fc afucosylated antibodies.
68. The population of antibodies according to any one of embodiments 62 to 65, comprising a relative frequency of 80 - 99.5 % of Fc galactosylated antibodies, a relative frequency of less than 20 % of Fc afucosylated antibodies, and a relative frequency of 50 % to 90 % of Fc sialylated antibodies.
69. The population of antibodies according to any one of embodiments 62 to 65, comprising a relative frequency of 80 - 99.5 % of Fc galactosylated antibodies, a relative frequency of 7 - 16 % of Fc afucosylated antibodies, and a relative frequency of 50 % to 90 % of Fc sialylated antibodies.
70. The population of antibodies according to any one of embodiments 62 to 69, wherein the population of antibodies is devoid of antibodies comprising a bisecting N-acetylglucosamine branch.
71. A method for the production of a population of galactoengineered recombinant antibodies, comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 70 % of Fc bi-galactosylated antibodies and a relative frequency of less than 20 % of Fc afucosylated antibodies.
72. The method according to embodiment 71, wherein the population of said galactoengineered recombinant antibody comprises a relative frequency of 80 - 95 % of Fc bi-galactosylated antibodies and a relative frequency of less than 20 % of Fc afucosylated antibodies.
73. The method according to embodiment 71, wherein the population of said galactoengineered recombinant antibody comprises a relative frequency of 80 - 95 % of Fc bi-galactosylated antibodies and a relative frequency of 7 - 16 % of Fc afucosylated antibodies.
74. The method according to embodiment 71, wherein the population of said galactoengineered recombinant antibody comprises a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
75. The method according to embodiment 71, wherein the population of said galactoengineered recombinant antibody comprises a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
76. The method according to any one of embodiments 71 to 75, wherein the population of said galactoengineered recombinant antibody is devoid of antibodies comprising a bi-secting N-acetylglucosamine branch.
77. The method according to any one of embodiments 71 to 76, wherein the Fab region of the antibody does not comprise the following amino acid sequences: Asn-X-Ser and Asn-X-Thr, wherein X is an amino acid except proline, Ser is serine and Thr is threonine.
78. The method according to any one of embodiments 71 to 77, wherein the antibody is a monoclonal full length antibody of human IgG1 isotype.
79. The method according to any one of embodiments 71 to 78, wherein the antibody is a humanized antibody.
80. The method according to any one of embodiments 71 to 79, wherein the antibody is an *in vivo* galactoengineered antibody.
81. The method according to any one of embodiments 71 to 80, wherein the antibody is selected from the group consisting of trastuzumab, rituximab, pertuzumab and obinutuzumab.
82. The method according to any one of embodiments 71 to 81, wherein the host cell is a eukaryotic cell.
83. The method according to any one of embodiments 71 to 81, wherein the host cell is a CHO cell.
84. The method according to any one of embodiments 71 to 83, wherein the host cell is not genetically engineered to improve or impair protein fucosylation and the method does not include the *in vivo* addition or *in vivo* cleavage of a fucose residue from the from the N-linked glycan structure of the antibodies.
85. A method for the production of a population of galactoengineered recombinant antibodies, comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 80 % of Fc galactosylated antibodies and a relative frequency of less than 20 % of Fc afucosylated antibodies.
86. The method according to embodiment 85, wherein the population of said galactoengineered recombinant antibody comprises a relative frequency of 80 - 95 % of Fc bi-galactosylated antibodies and a relative frequency of less than 20 % of Fc afucosylated antibodies.
87. The method according to embodiment 85, wherein the population of said galactoengineered recombinant antibody comprises a relative frequency of 80 - 95 % of Fc bi-galactosylated antibodies and a relative frequency of 7 - 16 % of Fc afucosylated antibodies.
88. The method according to embodiment 85, wherein the population of said galactoengineered recombinant antibody comprises a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
89. The method according to embodiment 85, wherein the population of said galactoengineered recombinant antibody comprises a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
90. The method according to any one of embodiments 85 to 89, wherein the population of said galactoengineered recombinant antibody is devoid of antibodies comprising a bi-secting N-acetylglucosamine branch.
91. The method according to any one of embodiments 85 to 90, wherein the Fab region of the antibody does not comprise the following amino acid sequences: Asn-X-Ser and Asn-X-Thr, wherein X is an amino acid except proline, Ser is serine and Thr is threonine.
92. The method according to any one of embodiments 85 to 91, wherein the antibody is a monoclonal full length antibody of human IgG1 isotype.
93. The method according to any one of embodiments 85 to 92, wherein the antibody is a humanized antibody.
94. The method according to any one of embodiments 85 to 93, wherein the antibody is an *in vivo* galactoengineered antibody.
95. The method according to any one of embodiments 85 to 94, wherein the antibody is selected from the group consisting of trastuzumab, rituximab, pertuzumab and obinutuzumab.
96. The method according to any one of embodiments 85 to 95, wherein the host cell is a eukaryotic cell.
97. The method according to any one of embodiments 85 to 96, wherein the host cell is a CHO cell.
98. The method according to any one of embodiments 85 to 97, wherein the host cell is not genetically engineered to improve or impair protein fucosylation and the method does not include the *in vivo* addition or *in vivo* cleavage of a fucose residue from the from the N-linked glycan structure of the antibodies.
99. A method for the production of a population of galactoengineered recombinant antibodies, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of galactoengineered recombinant antibodies, which comprises a relative frequency of at least 70 % of Fc bi-galactosylated antibodies and a relative frequency of less than 20 % of Fc afucosylated antibodies, and subsequent separation of the population of said galactoengineered recombinant antibodiesfrom said enzyme.
100. The method according to embodiment 99, wherein the population of said galactoengineered recombinant antibodies comprises a relative frequency of 80 - 95 % of Fc bi-galactosylated antibodies and a relative frequency of less than 20 % of Fc afucosylated antibodies.
101. The method according to embodiment 99, wherein the population of said galactoengineered recombinant antibodiescomprises a relative frequency of 80 - 95 % of Fc bi-galactosylated antibodies and a relative frequency of 7 - 16 % of Fc afucosylated antibodies.
102. The method according to embodiment 99, wherein the population of said galactoengineered recombinant antibodies comprises a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
103. The method according to embodiment 99, wherein the population of said galactoengineered recombinant antibodies comprises a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
104. The method according to any one of embodiments 99 to 103, wherein the population of said galactoengineered recombinant antibodies is devoid of antibodies comprising a bi-secting N-acetylglucosamine branch.
105. The method according to any one of embodiments 99 to 104, wherein the Fab region of the antibody does not comprise the following amino acid sequences: Asn-X-Ser and Asn-X-Thr, wherein X is an amino acid except proline, Ser is serine and Thr is threonine.
106. The method according to any one of embodiments 99 to 105, wherein the recombinant antibody is a monoclonal full length antibody of human IgG1 isotype.
107. The method according to any one of embodiments 99 to 106, wherein the recombinant antibody is a humanized antibody.
108. The method according to any one of embodiments 99 to 107, wherein the recombinant antibody is an *in vitro* galactoengineered antibody.
109. The method according to any one of embodiments 99 to 108, wherein the antibody is selected from the group consisting of trastuzumab, rituximab, pertuzumab and obinutuzumab.
110. The method according to any one of embodiments 99 to 109, wherein the host cell is a eukaryotic cell.
111. The method according to any one of embodiments 99 to 110, wherein the host cell is a CHO cell.
112. The method according to any one of embodiments 99 to 111, wherein the host cell is not genetically engineered to improve or impair protein fucosylation and the method does not include the *in vitro* addition or *in vitro* cleavage of a fucose residue from the from the N-linked glycan structure of the antibodies.
113. The method according to any one of embodiments 99 to 112, wherein step c) involves enzymatic treatment of said population of recombinant antibodies with galactosyltransferase in a solution of pH 6 - 7, at 30 - 35 °C for at least 150 min.
114. The method according to any one of embodiments 99 to 113, wherein step c) involves enzymatic treatment of said population of recombinant antibodies with galactosyltransferase in a solution of pH 6.3 - 6.8, at 31 - 34 °C for at least 150 min.
115. The method according to any one of embodiments 99 to 114, wherein step c) is performed in the presence of galactosyltransferase and UDP-galactose (Uridine diphosphate galactose).
116. The method according to any one of embodiments 99 to 115, wherein the population of recombinant antibodies obtained in step c) are subjected to
   d) an enzymatic treatment with sialyltransferase to obtain a population of galactoengineered recombinant antibodies, which comprises a relative frequency of up to 98 % of Fc sialylated antibodies (including mono-sialylated and bi-sialylated glycan structures) and subsequent separation of the population of sialylated antibodies from said sialyltransferase.
117. The method according to embodiment 116, wherein the enzymatic treatment with sialyltransferase is performed at the following conditions:
   i) sialyltransferase and the population of said galactoengineered recombinant antibodies obtained in step c) are contacted in solution for at least 150 min (in one embodiment for 2 - 120 h) at pH 6 - 8 (in one embodiment pH 6.5 - 7.5).
118. The method according to embodiment 116, the enzymatic treatment with sialyltransferase is performed at the following conditions:
   i) in a first step, sialyltransferase and the population of said galactoengineered recombinant antibodies obtained in step c) are contacted in solution for at least 150 min (in one embodiment for 2 - 120 h) at pH 6 - 8 (in one embodiment pH 6.5 - 7.5); and
   ii) in a second step, sialyltransferase and the population of antibodies obtained in step i) are contacted in solution for further at least 150 min (in one embodiment for 2 - 10 h) at pH 6 - 8 (in one embodiment pH 6.5 - 7.5).
119. The method according to any one of embodiments 116 to 118, wherein the treatment with sialyltransferase indicated in step i) or, optionally, i) - ii) is performed for a total time of up to 120 h.
120. The method according to any one of embodiments 116 to 119, wherein the antibody population obtained in step d) comprises a relative frequency of 80 % to 95 % of Fc bi-galactosylated antibodies, a relative frequency of less than 20 % of Fc afucosylated antibodies, and a relative frequency of 50 % to 90 % of Fc sialylated antibodies, wherein the population of antibodies is devoid of antibodies comprising a bisecting N-acetylglucosamine branch.
121. A method for the production of a population of galactoengineered recombinant antibodies comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of galactoengineered recombinant antibodies, which comprises a relative frequency of at least 80 % of Fc galactosylated antibodies, a relative frequency of less than 20 % of Fc afucosylated antibodies, and subsequent separation of the population of said galactoengineered recombinant antibodies from said enzyme.The method according to embodiment 85, wherein the population of said galactoengineered recombinant antibodies obtained in step c) comprises a relative frequency of 80 - 99.5 % of Fc galactosylated antibodies and a relative frequency of less than 20 % of Fc afucosylated antibodies.
122. The method according to embodiment 121, wherein the population of said galactoengineered recombinant antibodies obtained in step c) comprises a relative frequency of 80 - 99.5 % of Fc galactosylated antibodies and a relative frequency of 7 - 16 % of Fc afucosylated antibodies.
123. The method according to embodiment 121, wherein the population of said galactoengineered recombinant antibodies obtained in step c) comprises a relative frequency of 80 - 99.5 % of Fc galactosylated antibodies, a relative frequency of less than 20 % of Fc afucosylated antibodies, and a relative frequency of 50 % to 90 % of Fc sialylated antibodies.
124. The method according to embodiment 121, wherein the population of said galactoengineered recombinant antibodies obtained in step c) comprises a relative frequency of 80 - 99.5 % of Fc galactosylated antibodies, a relative frequency of 7 - 16 % of Fc afucosylated antibodies, and a relative frequency of 50 % to 90 % of Fc sialylated antibodies.
125. The method according to any one of embodiments 121 to 124, wherein the population of said galactoengineered recombinant antibodies obtained in step c) is devoid of antibodies comprising a bisecting N-acetylglucosamine branch.
126. The method according to any one of embodiments 121 to 125, wherein the Fab region of the antibody does not comprise the following amino acid sequences: Asn-X-Ser and Asn-X-Thr, wherein X is an amino acid except proline, Ser is serine and Thr is threonine.
127. The method according to any one of embodiments 121 to 126, wherein the antibody is a monoclonal full length antibody of human IgG1 isotype.
128. The method according to any one of embodiments 121 to 127, wherein the antibody is a humanized antibody.
129. The method according to any one of embodiments 121 to 128, wherein the antibody is an *in vitro* galactoengineered antibody.
130. The method according to any one of embodiments 121 to 129, wherein the antibody is selected from the group consisting of trastuzumab, rituximab, pertuzumab and obinutuzumab.
131. The method according to any one of embodiments 121 to 130, wherein the host cell is a eukaryotic cell.
132. The method according to any one of embodiments 121 to 131, wherein the host cell is a CHO cell.
133. The method according to any one of embodiments 121 to 132, wherein the host cell is not genetically engineered to improve or impair protein fucosylation and the method does not include the *in vitro* addition or *in vitro* cleavage of a fucose residue from the from the N-linked glycan structure of the antibodies.
134. The method according to any one of embodiments 121 to 133, wherein step c) involves enzymatic treatment of said population of antibodies with galactosyltransferase in a solution of pH 6 - 7, at 30 - 35 °C for at least 150 min.
135. The method according to any one of embodiments 121 to 134, wherein step c) involves enzymatic treatment of said population of antibodies with galactosyltransferase in a solution of pH 6.3 - 6.8, at 31 - 34 °C for at least 150 min.
136. The method according to any one of embodiments 121 to 135, wherein step c) is performed in the presence of galactosyltransferase and UDP-galactose (Uridine diphosphate galactose).
137. The method according to any one of embodiments 121 to 136, wherein the population of antibodies obtained in step c) are subjected to
   d) an enzymatic treatment with sialyltransferase to obtain an antibody population, which comprises a relative frequency of up to 98 % of Fc sialylated antibodies (including mono-sialylated and bi-sialylated glycan structures) and subsequent separation of the population of sialylated antibodies from said sialyltransferase.
138. The method according to embodiment 137, wherein the enzymatic treatment with sialyltransferase is performed at the following conditions:
   i) sialyltransferase and the population of antibodies obtained in step c) are contacted in solution for at least 150 min (in one embodiment for 2 - 120 h) at pH 6 - 8 (in one embodiment pH 6.5 - 7.5).
139. The method according to embodiment 137, the enzymatic treatment with sialyltransferase is performed at the following conditions:
   i) in a first step, sialyltransferase and the population of antibodies obtained in step c) are contacted in solution for at least 150 min (in one embodiment for 2 - 120 h) at pH 6 - 8 (in one embodiment pH 6.5 - 7.5); and
   ii) in a second step, sialyltransferase and the population of antibodies obtained in step i) are contacted in solution for further at least 150 min (in one embodiment for 2 - 10 h) at pH 6 - 8 (in one embodiment pH 6.5 - 7.5).
140. The method according to any one of embodiments 137 to 139, wherein the treatment with sialyltransferase indicated in step i) or, optionally, i) - ii) is performed for a total time of up to 120 h.
141. The method according to any one of embodiments 137 to 140, wherein the antibody population obtained in step d) comprises a relative frequency of 80 - 99.5 % of Fc galactosylated antibodies, a relative frequency of less than 20 % of Fc afucosylated antibodies, and a relative frequency of 50 % to 90 % of Fc sialylated antibodies, wherein the population of antibodies is devoid of antibodies comprising a bisecting N-acetylglucosamine branch.
142. A method for the production of a population of galactoengineered recombinant antibodies which specifically bind to HER2 and comprises a heavy chain variable domain of SEQ ID NO: 1 and a light chain variable domain of SEQ ID NO: 2, comprising the steps of, comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 70 % of Fc bi-galactosylated antibodies and a relative frequency of less than 20 % of Fc afucosylated antibodies.
143. A method for the production of a population of galactoengineered recombinant antibodies which specifically bind to CD20 and comprises a heavy chain variable domain of SEQ ID NO: 3 and a light chain variable domain of SEQ ID NO: 4, comprising the steps of, comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 70 % of Fc bi-galactosylated antibodies and a relative frequency of less than 20 % of Fc afucosylated antibodies.
144. A method for the production of a population of galactoengineered recombinant antibodies which specifically bind to HER2 and comprises a heavy chain variable domain of SEQ ID NO: 5 and a light chain variable domain of SEQ ID NO: 6, comprising the steps of, comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 70 % of Fc bi-galactosylated antibodies and a relative frequency of less than 20 % of Fc afucosylated antibodies.
145. A method for the production of a population of galactoengineered recombinant antibodies which specifically bind to CD20 and comprises a heavy chain variable domain of SEQ ID NO: 7 and a light chain variable domain of SEQ ID NO: 8, comprising the steps of, comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 70 % of Fc bi-galactosylated antibodies and a relative frequency of less than 20 % of Fc afucosylated antibodies.
146. A method for the production of a population of galactoengineered recombinant antibodies which specifically bind to HER2 and comprises a heavy chain variable domain of SEQ ID NO: 1 and a light chain variable domain of SEQ ID NO: 2, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 70 % of Fc bi-galactosylated antibodies and a relative frequency of less than 20 % of Fc afucosylated antibodies, and subsequent separation of the population of said galactoengineered recombinant antibodies from said enzyme.
147. A method for the production of a population of galactoengineered recombinant antibodies which specifically bind to CD20 and comprises a heavy chain variable domain of SEQ ID NO: 3 and a light chain variable domain of SEQ ID NO: 4, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 70 % of Fc bi-galactosylated antibodies and a relative frequency of less than 20 % of Fc afucosylated antibodies, and subsequent separation of the population of said galactoengineered recombinant antibodies from said enzyme.
148. A method for the production of a population of galactoengineered recombinant antibodies which specifically bind to HER2 and comprises a heavy chain variable domain of SEQ ID NO: 5 and a light chain variable domain of SEQ ID NO: 6, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 70 % of Fc bi-galactosylated antibodies and a relative frequency of less than 20 % of Fc afucosylated antibodies, and subsequent separation of the population of said galactoengineered recombinant antibodies from said enzyme.
149. A method for the production of a population of galactoengineered recombinant antibodies which specifically bind to CD20 and comprises a heavy chain variable domain of SEQ ID NO: 7 and a light chain variable domain of SEQ ID NO: 8, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 70 % of Fc bi-galactosylated antibodies and a relative frequency of less than 20 % of Fc afucosylated antibodies, and subsequent separation of the population of said galactoengineered recombinant antibodies from said enzyme.
150. The method according to any one of embodiments 142 to 149, wherein the population of said galactoengineered recombinant antibodies obtained in step c) comprises a relative frequency of 80 - 95 % of Fc bi-galactosylated antibodies and a relative frequency of less than 20 % of Fc afucosylated antibodies.
151. The method according to any one of embodiments 142 to 149, wherein the population of said galactoengineered recombinant antibodies obtained in step c) comprises a relative frequency of 80 - 95 % of Fc bi-galactosylated antibodies and a relative frequency of 7 - 16 % of Fc afucosylated antibodies.
152. The method according to any one of embodiments 142 to 149, wherein the population of said galactoengineered recombinant antibodies comprises a relative frequency of 80 - 95 % of Fc bi-galactosylated antibodies, a relative frequency of less than 20 % of Fc afucosylated antibodies, and a relative frequency of 50 % to 90 % of Fc sialylated antibodies.
153. The method according to any one of embodiments 142 to 149, wherein the population of said galactoengineered recombinant antibodies comprises a relative frequency of 80 - 95 % of Fc bi-galactosylated antibodies, a relative frequency of 7 - 16 % of Fc afucosylated antibodies, and a relative frequency of 50 % to 90 % of Fc sialylated antibodies.
154. The method according to any one of embodiments 142 to 153, wherein the population of said galactoengineered recombinant antibodies is devoid of antibodies comprising a bi-secting N-acetylglucosamine branch.
155. The method according to any one of embodiments 142 to 154, wherein the Fab region of the antibody does not comprise the following amino acid sequences: Asn-X-Ser and Asn-X-Thr, wherein X is an amino acid except proline, Ser is serine and Thr is threonine.
156. The method according to any one of embodiments 142 to 155, wherein the antibody is a monoclonal full length antibody of human IgG1 isotype.
157. The method according to any one of embodiments 142 to 156, wherein the antibody is a humanized antibody.
158. The method according to any one of embodiments 146 to 157, wherein the antibody is an *in vitro* galactoengineered antibody.
159. The method according to any one of embodiments 142 to 145 and 150 to 158, wherein the antibody is an *in vivo* galactoengineered antibody.
160. The method according to any one of embodiments 142 to 159, wherein the host cell is a eukaryotic cell.
161. The method according to any one of embodiments 142 to 159, wherein the host cell is a CHO cell.
162. The method according to any one of embodiments 142 to 161, wherein the host cell is not genetically engineered to improve or impair protein fucosylation and the method does not include the *in vitro* addition or *in vitro* cleavage of a fucose residue from the from the N-linked glycan structure of the antibodies.
163. The method according to any one of embodiments 146 to 162, wherein step c) involves enzymatic treatment of said population of recombinant antibodies with galactosyltransferase in a solution of pH 6 - 7, at 30 - 35 °C for at least 150 min.
164. The method according to any one of embodiments 146 to 163, wherein step c) involves enzymatic treatment of said population of recombinant antibodies with galactosyltransferase in a solution of pH 6.3 - 6.8, at 31 - 34 °C for at least 150 min.
165. The method according to any one of embodiments 146 to 164, wherein step c) is performed in the presence of galactosyltransferase and UDP-galactose (Uridine diphosphate galactose).
166. The method according to any one of embodiments 146 to 165, wherein the population of said galactoengineered recombinant antibodies obtained in step c) are subjected to
   d) an enzymatic treatment with sialyltransferase to obtain an antibody population, which comprises a relative frequency of up to 98 % of Fc sialylated antibodies (including mono-sialylated and bi-sialylated glycan structures) and subsequent separation of the population of sialylated antibodies from said sialyltransferase.
167. The method according to embodiment 166, wherein the enzymatic treatment with sialyltransferase is performed at the following conditions:
   i) sialyltransferase and the population of said galactoengineered recombinant antibodies obtained in step c) are contacted in solution for at least 150 min (in one embodiment for 2 - 120 h) at pH 6 - 8 (in one embodiment pH 6.5 - 7.5).
168. The method according to embodiment 166, the enzymatic treatment with sialyltransferase is performed at the following conditions:
   i) in a first step, sialyltransferase and the population of said galactoengineered recombinant antibodies obtained in step c) are contacted in solution for at least 150 min (in one embodiment for 2 - 120 h) at pH 6 - 8 (in one embodiment pH 6.5 - 7.5); and
   ii) in a second step, sialyltransferase and the population of antibodies obtained in step i) are contacted in solution for further at least 150 min (in one embodiment for 2 - 10 h) at pH 6 - 8 (in one embodiment pH 6.5 - 7.5).
169. The method according to any one of embodiments 166 to 168, wherein the treatment with sialyltransferase indicated in step i) or, optionally, i) - ii) is performed for a total time of up to 120 h.
170. The method according to any one of embodiments 166 to 169, wherein the antibody population obtained in step d) comprises a relative frequency of 80 % to 95 % of Fc bi-galactosylated antibodies, a relative frequency of less than 20 % of Fc afucosylated antibodies, and a relative frequency of 50 % to 90 % of Fc sialylated antibodies, wherein the population of antibodies is devoid of antibodies comprising a bisecting N-acetylglucosamine branch.
171. A method for the production of a population of galactoengineered recombinant antibodies, which specifically bind to HER2 and comprises a heavy chain variable domain of SEQ ID NO: 1 and a light chain variable domain of SEQ ID NO: 2,comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 80 % of Fc galactosylated antibodies and a relative frequency of less than 20 % of Fc afucosylated antibodies.
172. A method for the production of a population of galactoengineered recombinant antibodies, which specifically bind to CD20 and comprises a heavy chain variable domain of SEQ ID NO: 3 and a light chain variable domain of SEQ ID NO: 4,comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 80 % of Fc galactosylated antibodies and a relative frequency of less than 20 % of Fc afucosylated antibodies.
173. A method for the production of a population of galactoengineered recombinant antibodies, which specifically bind to HER2 and comprises a heavy chain variable domain of SEQ ID NO: 5 and a light chain variable domain of SEQ ID NO: 6,comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 80 % of Fc galactosylated antibodies and a relative frequency of less than 20 % of Fc afucosylated antibodies.
174. A method for the production of a population of galactoengineered recombinant antibodies, which specifically bind to CD20 and comprises a heavy chain variable domain of SEQ ID NO: 7 and a light chain variable domain of SEQ ID NO: 8,comprising the steps of
   a) recombinantly producing an antibody of IgG1 isotype in a host cell, which comprises nucleic acid molecules encoding the antibody, to obtain a population of a recombinant antibody,
   b) genetically engineering the host cell to improve protein galactosylation such that a population of said galactoengineered recombinant antibody is obtained, which comprises a relative frequency of at least 80 % of Fc galactosylated antibodies and a relative frequency of less than 20 % of Fc afucosylated antibodies.
175. A method for the production of a population of galactoengineered recombinant antibodies, which specifically bind to HER2 and comprises a heavy chain variable domain of SEQ ID NO: 1 and a light chain variable domain of SEQ ID NO: 2, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 80 % of Fc galactosylated antibodies, a relative frequency of less than 20 % of Fc afucosylated antibodies, and subsequent separation of the population of said galactoengineered recombinant antibodies from said enzyme.
176. A method for the production of a population of galactoengineered recombinant antibodies, which specifically bind to CD20 and comprises a heavy chain variable domain of SEQ ID NO: 3 and a light chain variable domain of SEQ ID NO: 4, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 80 % of Fc galactosylated antibodies, a relative frequency of less than 20 % of Fc afucosylated antibodies, and subsequent separation of the population of said galactoengineered recombinant antibodies from said enzyme.
177. A method for the production of a population of galactoengineered recombinant antibodies, which specifically bind to HER2 and comprises a heavy chain variable domain of SEQ ID NO: 5 and a light chain variable domain of SEQ ID NO: 6, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 80 % of Fc galactosylated antibodies, a relative frequency of less than 20 % of Fc afucosylated antibodies, and subsequent separation of the population of said galactoengineered recombinant antibodies from said enzyme.
178. A method for the production of a population of galactoengineered recombinant antibodies, which specifically bind to CD20 and comprises a heavy chain variable domain of SEQ ID NO: 7 and a light chain variable domain of SEQ ID NO: 8, comprising the steps of
   a) recombinantly producing an antibody of IgG1 isotype in a host cell, which comprises nucleic acid molecules encoding the antibody, to obtain a population of a recombinant antibody,
   b) isolating said population of the recombinant antibody,
   c) enzymatically treating said population of recombinant antibodies with galactosyltransferase to obtain a population of said galactoengineered recombinant antibodies, which comprises a relative frequency of at least 80 % of Fc galactosylated antibodies, a relative frequency of less than 20 % of Fc afucosylated antibodies, and subsequent separation of the population of said galactoengineered recombinant antibodies from said enzyme.
179. The method according to any one of embodiments 171 to 178, wherein the population of said galactoengineered recombinant antibodies comprises a relative frequency of 80 - 99.5 % of Fc galactosylated antibodies and a relative frequency of less than 20 % of Fc afucosylated antibodies.
180. The method according to any one of embodiments 171 to 179, wherein the population of said galactoengineered recombinant antibodies comprises a relative frequency of 80 - 99.5 % of Fc galactosylated antibodies and a relative frequency of 7 - 16 % of Fc afucosylated antibodies.
181. The method according to any one of embodiment 171 to 180, wherein the population of said galactoengineered recombinant antibodies comprises a relative frequency of 80 - 99.5 % of Fc galactosylated antibodies, a relative frequency of less than 20 % of Fc afucosylated antibodies, and a relative frequency of 50 % to 90 % of Fc sialylated antibodies.
182. The method according to any one of embodiments 171 to 181, wherein the population of said galactoengineered recombinant antibodies comprises a relative frequency of 80 - 99.5 % of Fc galactosylated antibodies, a relative frequency of 7 - 16 % of Fc afucosylated antibodies, and a relative frequency of 50 % to 90 % of Fc sialylated antibodies.
183. The method according to any one of embodiments 171 to 182, wherein the population of said galactoengineered recombinant antibodies is devoid of antibodies comprising a bisecting N-acetylglucosamine branch.
184. The method according to any one of embodiments 171 to 183, wherein the Fab region of the antibody does not comprise the following amino acid sequences: Asn-X-Ser and Asn-X-Thr, wherein X is an amino acid except proline, Ser is serine and Thr is threonine.
185. The method according to any one of embodiments 171 to 184, wherein the antibody is a monoclonal full length antibody of human IgG1 isotype.
186. The method according to any one of embodiments 171 to 185, wherein the recombinant antibody is a humanized antibody.
187. The method according to any one of embodiments 175 to 186, wherein the recombinant antibody is an *in vitro* galactoengineered antibody.
188. The method according to any one of embodiments 171 to 174 and 179 to 186, wherein the recombinant antibody is an *in vivo* galactoengineered antibody.
189. The method according to any one of embodiments 171 to 188, wherein the host cell is a eukaryotic cell.
190. The method according to any one of embodiments 171 to 189, wherein the host cell is a CHO cell.
191. The method according to any one of embodiments 171 to 190, wherein the host cell is not genetically engineered to improve or impair protein fucosylation and the method does not include the *in vitro* addition or *in vitro* cleavage of a fucose residue from the from the N-linked glycan structure of the antibodies.
192. The method according to any one of embodiments 175 to 191, wherein step c) involves enzymatic treatment of said population of recombinant antibodies with galactosyltransferase in a solution of pH 6 - 7, at 30 - 35 °C for at least 150 min.
193. The method according to any one of embodiments 175 to 192, wherein step c) involves enzymatic treatment of said population of recombinant antibodies with galactosyltransferase in a solution of pH 6.3 - 6.8, at 31 - 34 °C for at least 150 min.
194. The method according to any one of embodiments 175 to 193, wherein step c) is performed in the presence of galactosyltransferase and UDP-galactose (Uridine diphosphate galactose).
195. The method according to any one of embodiments 175 to 194, wherein the population of said galactoengineered recombinant antibodies obtained in step c) are subjected to
   d) an enzymatic treatment with sialyltransferase to obtain an antibody population, which comprises a relative frequency of up to 98 % of Fc sialylated antibodies (including mono-sialylated and bi-sialylated glycan structures) and subsequent separation of the population of sialylated antibodies from said sialyltransferase.
196. The method according to embodiment 195, wherein the enzymatic treatment with sialyltransferase is performed at the following conditions:
   i) sialyltransferase and the population of said galactoengineered recombinant antibodies obtained in step c) are contacted in solution for at least 150 min (in one embodiment for 2 - 120 h) at pH 6 - 8 (in one embodiment pH 6.5 - 7.5).
197. The method according to embodiment 195, the enzymatic treatment with sialyltransferase is performed at the following conditions:
   i) in a first step, sialyltransferase and the population of said galactoengineered recombinant antibodies obtained in step c) are contacted in solution for at least 150 min (in one embodiment for 2 - 120 h) at pH 6 - 8 (in one embodiment pH 6.5 - 7.5); and
   ii) in a second step, sialyltransferase and the population of antibodies obtained in step i) are contacted in solution for further at least 150 min (in one embodiment for 2 - 10 h) at pH 6 - 8 (in one embodiment pH 6.5 - 7.5).
198. The method according to any one of embodiments 195 to 197, wherein the treatment with sialyltransferase indicated in step i) or, optionally, i) - ii) is performed for a total time of up to 120 h.
199. The method according to any one of embodiments 195 to 198, wherein the antibody population obtained in step d) comprises a relative frequency of 80 - 99.5 % of Fc galactosylated antibodies, a relative frequency of less than 20 % of Fc afucosylated antibodies, and a relative frequency of 50 % to 90 % of Fc sialylated antibodies, wherein the population of antibodies is devoid of antibodies comprising a bisecting N-acetylglucosamine branch.
200. A pharmaceutical composition comprising a population of galactoengineered recombinant antibodies of IgG1 isotype according to any one of embodiments 1 to 70.
201. A pharmaceutical composition comprising a population of galactoengineered recombinant antibodies of IgG1 isotype obtained by a method according to any one of embodiments 71 to 199.
202. The pharmaceutical composition according to embodiment 200 or 201, wherein the galactoengineeredantibody is formulated in combination with at least one pharmaceutically acceptable carrier.
203. A method of treatment of a patient suffering from a disease by administering a population of galactoengineeredrecombinant antibodies of IgG1 isotype according to any one of embodiments 1 to 70 to the patient in the need of such treatment.
204. A method of treatment of a patient suffering from a disease by administering a population of galactoengineeredrecombinant antibodies of IgG1 isotype obtained by a method according to any one of embodiments 71 to 199 to the patient in the need of such treatment.
205. The method according to any one of embodiments 203 or 204, wherein the disease is selected from the group consisting of cancer, inflammatory disorder and autoimmune disorder.
206. The method according to any one of embodiments 71 to 199 for improving ADCC mediated by said population of recombinant antibodies of IgG1 isotype.
207. Use of the method according to any one of embodiments 65 to 218 for improving the binding affinity of recombinant antibodies to Fc-gammaRIIa and Fc-gammaRIIIa.
208. Use of a population of galactoengineeredrecombinant antibodies according to any one of embodiments 1 to 70 or obtained by a method according to any one of embodiments 71 to 199 for mediating ADCC.
209. Use of a population of galactoengineeredrecombinant antibodies according to any one of embodiments 1 to 70 or obtained by a method according to any one of embodiments 71 to 199 for recruiting and / or activating natural killer cells.
210. Use of a population of galactoengineeredrecombinant antibodies according to any one of embodiments 1 to 70 or obtained by a method according to any one of embodiments 71 to 199 for activation of Fc-gammaR-comprising effector cells.
211. The population of galactoengineeredrecombinant antibodies according to any one of embodiments 1 to 70 or obtained by a method according to any one of embodiments 71 to 199 for use as a medicament.
212. The population of galactoengineeredrecombinant antibodies according to any one of embodiments 1 to 70 or obtained by a method according to any one of embodiments 71 to 199 for use in the treatment of a disease selected from the group consisting of cancer, inflammatory disorder and autoimmune disorder.
213. The population of galactoengineeredrecombinant antibodies obtained by a method according to any one of embodiments 71 to 199.

**DESCRIPTION OF THE AMINO ACID SEQUENCES**

| | |
|---|---|
| **SEQ ID NO:1** | Heavy chain variable domain of antibody trastuzumab |
| | |
| **SEQ ID NO:2** | Light chain variable domain of antibody trastuzumab |
| | |
| **SEQ ID NO:3** | Heavy chain variable domain of antibody rituximab |
| | QVQLQQPGAELVKPGASVKMSCKASGYTFTSYNMHWVKQTPGR GLEWIGAIYPGNGDTSYNQKFKGKATLTADKSSSTAYMQLSSL TSEDSAVYYCARSTYYGGDWYFNVWGAGTTVTVS |
| **SEQ ID NO:4** | Light chain variable domain of antibody rituximab |
| | QIVLSQSPAILSASPGEKVTMTCRASSSVSYIHWFQQKPGSSP KPWIYATSNLASGVPVRFSGSGSGTSYSLTISRVEAEDAATYY CQQWTSNPPTFGGGTKLEIKR |
| **SEQ ID NO:5** | Heavy chain variable domain of antibody pertuzumab |
| | EVQLVESGGGLVQPGGSLRLSCAASGFTFTDYTMDWVRQAPGK GLEWVADVNPNSGGSIYNQRFKGRFTLSVDRSKNTLYLQMNSL RAEDTAVYYCARNLGPSFYFDYWGQGTLVTVSSA |
| **SEQ ID NO:6** | Light chain variable domain of antibody pertuzumab |
| | DIQMTQSPSSLSASVGDRVTITCKASQDVSIGVAWYQQKPGKA PKLLIYSASYRYTGVPSRFSGSGSGTDFTLTISSLQPEDFATY YCQQYYIYPYTFGQGTKVEI |
| **SEQ ID NO:7** | Heavy chain variable domain of antibody obinutuzumab |
| | QVQLVQSGAEVKKPGSSVKVSCKASGYAFSYSWINWVRQAPGQ GLEWMGRIFPGDGDTDYNGKFKGRVTITADKSTSTAYMELSSL RSEDTAVYYCARNVFDGYWLVYWGQGTLVTVSSA |
| **SEQ ID NO:8** | Light chain variable domain of antibody obinutuzumab |
| | DIVMTQTPLSLPVTPGEPASISCRSSKSLLHSNGITYLYWYLQ KPGQSPQLLIYQMSNLVSGVPDRFSGSGSGTDFTLKISRVEAE DVGVYYCAQNLELPYTFGGGTK |
| **SEQ ID NO: 9** | Sialyltransferase ST6 (Roche, product no. 07012250103) |
| | EASFQVWNKDSSSKNLIPRLQKIWKNYLSMNKYKVSYKGPGPG IKFSAEALRCHLRDHVNVSMVEVTDFPFNTSEWEGYLPKESIR TKAGPWGRCAVVSSAGSLKSSQLGREIDDHDAVLRFNGAPTAN FQQDVGTKTTIRLMNSQLVTTEKRFLKDSLYNEGILIVWDPSV YHSDIPKWYQNPDYNFFNNYKTYRKLHPNQPFYILKPQMPWEL WDILQEISPEEIQPNPPSSGMLGIIIMMTLCDQVDIYEFLPSK RKTDVCYYYQKFFDSACTMGAYHPLLYEKNLVKHLNQGTDEDI YLLGK |
| **SEQ ID NO: 10** | Sialyltransferase ST6 |
| | LQKIWKNYLSMNKYKVSYKGPGPGIKFSAEALRCHLRDHVNVS MVEVTDFPFNTSEWEGYLPKESIRTKAGPWGRCAVVSSAGSLK SSQLGREIDDHDAVLRFNGAPTANFQQDVGTKTTIRLMNSQLV TTEKRFLKDSLYNEGILIVWDPSVYHSDIPKWYQNPDYNFFNN YKTYRKLHPNQPFYILKPQMPWELWDILQEISPEEIQPNPPSS GMLGIIIMMTLCDQVDIYEFLPSKRKTDVCYYYQKFFDSACTM GAYHPLLYEKNLVKHLNQGTDEDIYLLGKATLPGFRTIHC |

### EXAMPLES

The following examples are provided to aid the understanding of the present invention, the true scope of which is set forth in the appended claims. It is understood that modifications can be made in the procedures set forth without departing from the spirit of the invention.

### Example 1:

### Preparation of highly galactosylated IgG1 antibodies (trastuzumab) by in vitro glycoengineering and identification of glycostructures

In a first experiment, trastuzumab (mAb1) obtained from four different production batches was enzymatically hypergalactosylated.

For generation of such highly galactosylated (hypergalactosylated) mAb trastuzumab, bovine beta-1,4-Galactosyltransferase (Roche) was added to the antibodies. Reaction buffer (20 mM MnCl, 10 mM UDP-Gal, 100 mM MES buffer, pH 6.5) and H₂O were added to achieve a concentration between 3 and 15 mg mAb /ml. Samples were incubated at temperatures ranging from 32 to 37°C. Further amounts of enzyme were added either following 24 h incubation (22 mU) or 48 h incubation (44 mU). The reaction was stopped by buffer exchange to 25 mM Na-citrate, and pH adjusted to pH 5.5 using MabSelect Sure protein A columns. Final mAb concentration ranged from 2 to 15 mg/ml.

For analysis of the glycostructures, 150 µg mAb were subjected to buffer exchange to phosphate buffer (10 mM, pH7.2). Followed by incubation with 2 µl PNGase F (500000 U/ml, BioLabs) at 45°C for 1h and 2-AB labeling at 65°C for 2 h (Signal 2-AB labeling Kit, Glyko, GKK404). Analysis was done via NP-UHPLC on a HILIC column (Waters, BEH Glycan 1.7 µm, 2.1 x 150 mm; 45 min gradient).

A comparison of the glycostructures of the untreated original material and the galactosyltransferase-treated population is indicated in table 1.

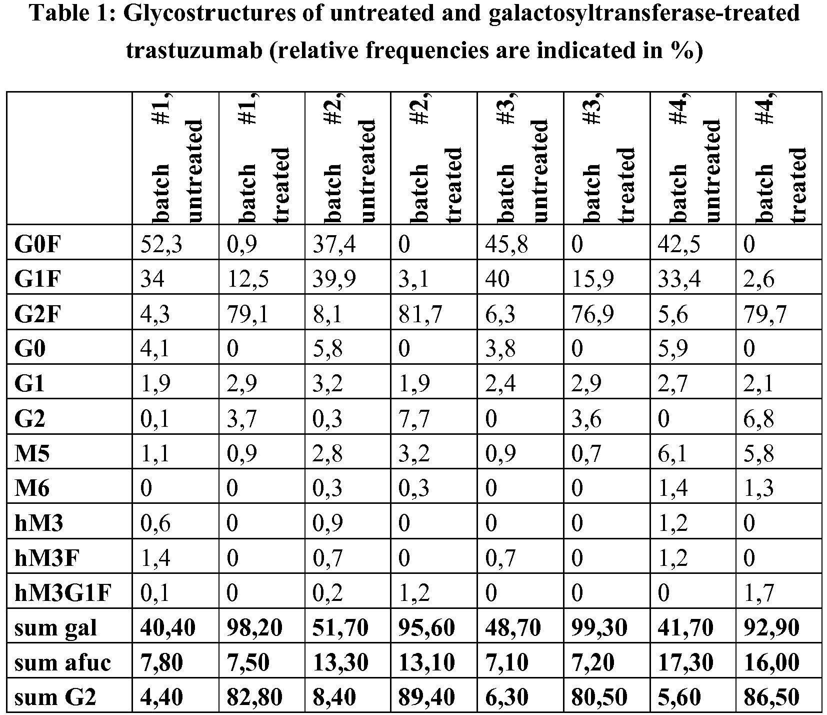

### Example 2:

### Assessment of ADCC mediated by highly galactosylated IgG1 antibodies

The ADCC mediated by the antibodies was analyzed in a NK cell-line based *in vitro* ADCC assay.

For this analysis, recombinant effector cells expressing human Fc-gammaRIIIa were generated and cultured as previously described (Schnueriger, A. et al. Mol Immunol 48, 1512-1517 (2011), included by reference herein). The target cell lines were purchased from American Type Culture Collection (ATCC). The various target cell lines were labeled with BATDA ligand (Perkin Elmer) according to the recommendations given by the supplier. Effector cells and labeled target cells were mixed in growth medium and distributed in 96-well micro titer plates. Antibody samples were diluted in growth medium and added to the effector-target-cell mix. Assay plates were incubated in a humidified incubator at 37°C/5 % CO₂. After incubation, plates were centrifuged and supernatants from each well were transferred to a white 96-well plate. Two hundred microliters of europium solution (Perkin Elmer) was added to each well, followed by a short incubation on a plate shaker. Controls for spontaneous and maximum release were prepared following the instructions given by the supplier of the BATDA ligand (Perkin Elmer). Time-resolved fluorescence was measured in RFU (Relative Fluorescence Units) using excitation at 345 nm; emission at 615 nm. Readings were performed on a Spectramax M5 plate reader (Molecular Devices). Specific toxicity values were calculated as follows: 100 % x (specific release - spontaneous release)/(maximum release - spontaneous release). For relative ADCC calculation, % specific toxicity was plotted against antibody concentration and relative ADCC was determined by full curve parallel line analysis (Gottschalk, P.G. & Dunn, J.R. J Biopharm Stat 15, 437-463 (2005)).

Modified samples were analyzed side by side with reference material, starting material and mock-treated controls. Each sample was analyzed in five independent experiments. The mock-treated controls (referred to as control), prepared in parallel to the modified samples in the absence of modifying enzyme, showed no changes in ADCC compared to starting material (data not shown).

Fig. 2A shows representative dose-dependent ADCC mediated by hypergalactosylated trastuzumab (mAb1) samples (dashed lines, white squares) and the untreated control mAb samples (solid lines, black circles). The maximum target cell lysis at saturating antibody concentrations (approximately 1000 ng/ml) reached comparable levels irrespective of sample treatment. Differences between controls and hypergalactosylated material were observed with respect to EC50 values which were lower for hypergalactosylated material. Thus, hypergalactosylated trastuzumab samples consistently elicited increased ADCC relative to their mock treated controls.

To determine whether the influence of galactose on ADCC activity was a more common phenomenon, hypergalactosylated samples were generated using a set of antibodies to different targets. The *in vitro* NK cell-based assay system was adapted to these different antibodies, and the ADCC activity of the glyco-modified samples and their associated controls, was compared relative to the respective reference standards for each antibody in three independent experiments. Similar to the dose-response curves observed with trastuzumab, the hypergalactosylated samples displayed lower EC50 values compared to respective control material, while maximum ADCC at saturating concentrations remained unchanged in all cases (data not shown). The quantitative results expressed in ADCC, relative to reference material are illustrated in Figure 2B. Upon hypergalactosylation, the relative ADCC of rituximab (mAb2) batches # 1, 2 and 3 increased from 55 %, 96 % and 111 % for the controls (Figure 2B, grey bars) to 85 %, 113 % and 123 %, for the hypergalactosylated samples (Figure 2B, white bars). In the case of pertuzumab (mAb3), the relative ADCC of batches #1 and 2 increased from 64% and 53 % for the control samples (Figure 2B, grey bars) to 75 % and 71 % (Figure 2B, white bars), for the hypergalactosylated samples. mAb4 batches # 1, 2 and 3 showed increases in ADCC from 48 %, 97 % and 90 % (Figure 2B, grey bars) for the controls, to 58 %, 112 % and 98 % (Figure 2B, white bars) for the hypergalactosylated samples. In general, the tendency for hypergalactosylation to enhance ADCC activity was observed with all 12 antibody batches tested for mAb1-4.

Given the predominant impact of afucose on ADCC, the question arises whether higher galactose levels could modulate ADCC of mAb containing high levels of afucosylation. To address this, afucosylated mAb4 (mAb4 afu) and afucosylated obinutuzumab (mAb5 afu) were galactosylated enzymatically and compared to their associated controls (Figure 2B). The dose response curves of control material and hypergalactosylated material were largely overlapping (data not shown). Quantitatively, relative ADCC mediated by afucosylated mAb4 (100 %) and afucosylated obinutuzumab (107 %) control material (Figure 2B, grey bars) was comparable to the ADCC of hypergalacosylated material (101 % and 108 % for afucosylated mAb4 and afucosylated obinutuzumab, respectively. See figure 2B, white bars). Thus, upon hypergalactosylation, the ADCC of highly afucosylated antibodies remained unchanged.

Afucosylated mAb4 variant was produced using a *FUT-8* -/- CHO expression system (mAb4 afu), while afucosylated variant of trastuzumab (mAb1 afu) and afucosylated variant of obinutuzumab (mAb5 afu) were produced from cells over-expressing GnT-III (beta-1,4-N-Acetylglucosaminyltransferase III).

### Example 3:

### Preparation of IgG1 antibody (trastuzumab) populations with different N-linked glycan structures by in vitro glycoengineering

In a second experiment, trastuzumab obtained from a standard production batch was subjected to *in vitro* glycoengineering to produce different trastuzumab populations with varying glycan-profiles. A schematic workflow of the *in vitro* glycoengineering procedure is depicted in Fig. 3.

### Agalactosylated glycan species ("aGal"):

A population of trastuzumab mAbs comprising a high relative frequency of agalactosylated glycan structures (e.g. G0, G0F) was prepared by treating the mAbs with galactosidase. Briefly, for preparation of agalactosylated antibodies 5 µl β(1-4)-Galactosidase (Prozyme, GKX-5014) was added to 1 mg of trastuzumab (10 mU enzyme/mg antibody) and incubated at 37 °C for 24 hours. The enzyme-treated mAb population was purified from the free enzyme via Protein A chromatography subsequently.

### Bi-galactosylated glycan species ("biGal"):

A population of trastuzumab mAbs comprising a high relative frequency of bi-galactosylated glycan structures (e.g. G2, G2, G2S1 and G2S2 and the corresponding fucosylated species) was prepared by treating the mAbs with galactosyltransferase according to the method described in Example 1. Briefly, 1 g trastuzumab (c = 25 mg/ml) was mixed with 157 ml reaction buffer (10 mM UDP-Gal, 20 mM MnCl₂, 100 mM MES pH 6.5). beta-(1-4)-Galactosyltransferase (G5507-25U) from Sigma was diluted with dH₂O to a concentration of 10 U/ml. 4.6 ml galactosyltransferase was added to the sample at incubation start und further 2.3 ml after 2 and 3 days. The total incubation time was 4 days at 32 °C. The enzyme-treated sample was subsequently purified by Protein A chromatography.

### Bi-galactosylated, mono-sialylated glycan species ("biGal/monoSia"):

To prepare a population of trastuzumab mAbs comprising a high relative frequency of bi-galactosylated and mono-sialylated glycan structures (e.g. G2S1 and G2S1F), the obtained population of bi-galactosylated glycan species (which was prepared as described above) was subjected to an enzymatic treatment with sialyltransferase. Briefly, to achieve mono-sialylation of 500 mg of the bi-galactosylated trastuzumab sample as described above (c = 8.1 mg/ml) 50 mg ST6 and 25 ml of an aqueous CMP-NANA solution with a concentration of 10 mg/ml were added. The sample was incubated for 24 h at 37 °C before being purified by Protein A purification.

### Bi-galactosylated, bi-sialylated glycan species ("biGal/biSia"):

To prepare a population of trastuzumab mAbs comprising a high relative frequency of bi-galactosylated and bi-sialylated glycan structures (e.g. G2S2 and G2S2F), the obtained population of bi-galactosylated and mono-sialylated glycan species (which was prepared as described above) was subjected to another enzymatic treatment with sialyltransferase, using sialyltransferase ST6 (Roche, amino acid sequence according to SEQ ID NO:9). In brief, for further increase of the degree of sialylation 100 mg of the bi-galactosylated and mono-sialylated trastuzumab sample was mixed with 3 ml of the aqueous CMP-NANA solution and 10 mg ST6 (Roche, prod.nr. 07012250103). The sample was incubated for 7 h at 37 °C before being purified by subsequent Protein A purification.

### Example 4:

### Glycan analysis of IgG1 antibody (trastuzumab) populations with different N-linked glycan structures

For analysis of the glycan structures of the individual glycoengineered trastuzumab samples of Example 3 (by the same method as described in Example 1), 150 µg of the respective mAbs were subjected to buffer exchange to phosphate buffer (10 mM, pH 7.2), followed by incubation with 2 µl PNGase F (500000 U/ml, BioLabs) at 45°C for 1 h and 2-AB labeling at 65°C for 2 h with subsequent purification (Signal 2-AB labeling Kit, Glyko, GKK404). The labeled glycans were separated by hydrophilic interaction (BEH glycan column, 1.7 µm, 2.1 x 150 mm, Waters) liquid chromatography (45 min gradient) and the fluorescence signal was detected at 420 nm (excitation wavelength at 330 nm).

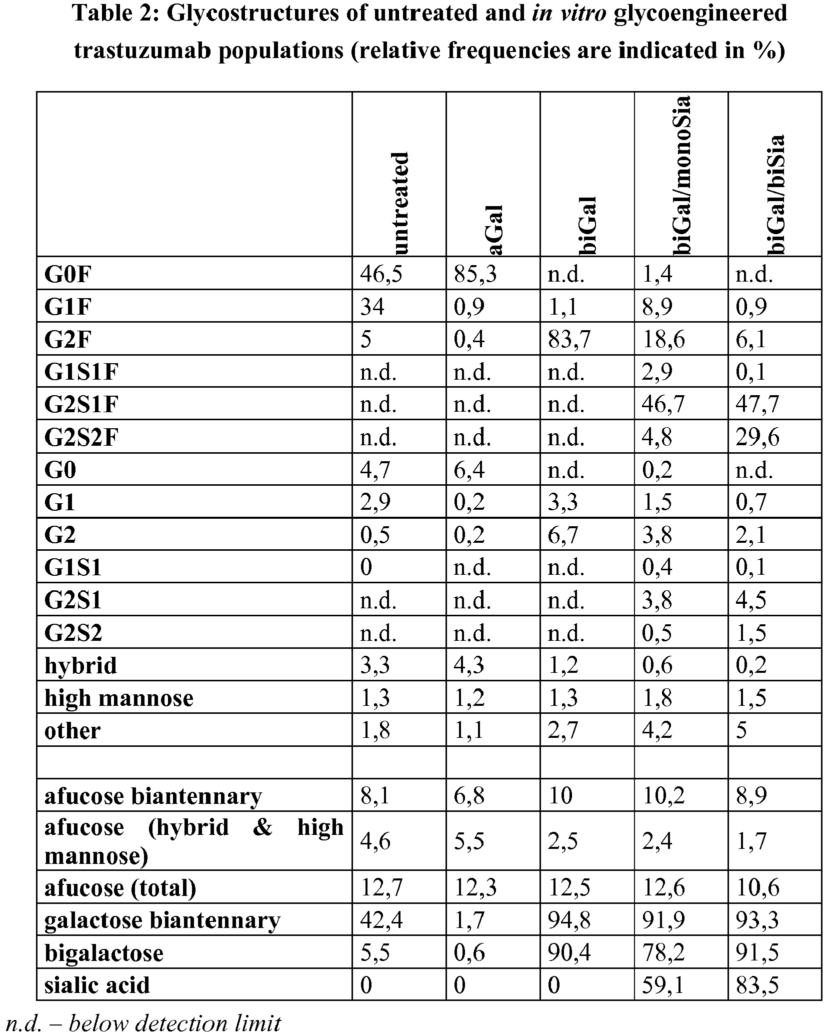

As can be seen from table 2, the respective *in vitro* glycoengineering resulted in an improved fraction of the desired glycan species when compared to the untreated sample. In the agalactosylated sample derived by galactosidase treatment, more than 85 % of the glycan species were agalactosylated. In the sample provided to include a high frequency of bi-galactosylated trastuzumab antibodies, more than 80 % of the glycan species were bi-galactosylated. In the sample prepared to include an increased amount of mono-sialylated trastuzumab antibodies, more than 45 % of the glycan species were mono-sialylated with an additional fraction of about 5 % of bi-sialylated IgG1. In the last sample prepared to include an increased amount of bi-sialylated trastuzumab antibodies, the fraction of bi-sialylated IgG1 was further increased to about 30 % with about the same fraction of mono-sialylated antibodies present in the sample.

As the distinct samples can be considered to comprise a rather homogeneous glycan profile, each of the samples was analyzed with respect to its influence on Fc-gammaR binding and mediation of ADCC.

### Example 5:

### Assessment of Fc-gammaR binding mediated by different glycan species of IgG1 antibodies (trastuzumab) - surface plasmon resonance

For analysis of Fc-gammaR binding by surface plasmon resonance (SPR), a Biacore® T100 system (GE Healthcare) was used. For interaction analysis of Fc-gammaRs and IgG1 glycovariants, an anti-His capturing antibody (GE Healthcare) was injected to achieve a level of 12,000 resonance units (RU). Immobilization of the capturing antibody was performed on a CM5 chip using the standard amine coupling kit (GE Healthcare) at pH 4.5. Fc-gammaRIa, Fc-gammaRIIa and Fc-gammaRIIIa were captured at a concentration of 200 nM with a pulse of 60 sec at a flow rate of 10 µl/min. Subsequently trastuzumab glycovariants were applied at a concentration of 300 nM and a flow rate of 30 µl/min for 60 sec. The dissociation phase was monitored for 180 sec. The surface was regenerated by a 60 sec washing step with a 10 mM Glycine pH 1.5 at a flow rate of 30 µl/min. All experiments were carried out in HBS-N buffer (10 mM HEPES, pH 7.4, 150 mM NaCl). The Biacore T100 evaluation software 2.0.3 was used for data evaluation.

Results are shown in Fig. 4. Indicated (y-axis) is the binding intensity relative to the untreated control sample (which is therefore normalized to 100 % binding to the respective receptor). The data demonstrate that agalactosylation of trastuzumab leads to decreased Fc-gammaRIa, IIa and IIIa binding (significant decrease for Fc-gammaRIIa and IIIa). The trastuzumab samples with a high frequency of bi-galactosylated glycan species exhibited improved binding to Fc-gammaRIIa and Fc-gammaRIIIa.

### Example 6:

### Assessment of Fc-gammaR binding mediated by different glycan species of IgG1 antibodies (trastuzumab) - affinity chromatography

For analysis of Fc-gammaR binding by affinity chromatography, biotinylated human Fc-gammaRIIa or Fc-gammaRIIIa_V158 were incubated, respectively, with streptavidin sepharose for 2 hours upon mild shaking. The respective Fc-gamma-receptor-derivatized sepharose was packed in a Tricorn 5/50 Column housing (inner diameter 5 mm xlength 50 mm, GE Healthcare) and the affinity column was equilibrated with 20 mM Tris, 150 mM NaCl pH 8.0 (Fc-gammaRIIa) or 20 mM Sodium Citrate, 150 mM NaCl pH 6.0 (Fc-gammaRIIIa) at flow rate of 0.5 ml/min using Äkta explorer or Dionex Summit system. The antibody samples containing 50 to 100 µg in equilibration buffer were applied to the respective column. Subsequently, columns were washed with 5 column volumes of equilibration buffer. The samples were eluted with a linear pH gradient of 15 column volumes with 20 mM Citrat, 150 mM NaCl pH 4.0 (Fc-gammaRIIa) or pH 3.0 (Fc-gammaRIIIa) respectively. The experiments were carried out at room temperature. The elution profile was obtained by continuous measurement of the absorbance at 280 nm.

Results are shown in Fig. 5. Indicated is the retention time of the respective samples. For Fc-gammaRIIIa binding, retention times for the fucosylated glycan species (early eluting peak in affinity chromatography) and the (partially) afucosylated glycan species (late eluting peak in affinity chromatography) is indicated. In line with the SPR data, these data demonstrate that agalactosylation decreases Fc-gammaRIIa and IIIa binding, while bi-galactosylation improves the binding of trastuzumab to said receptors. The effect can be observed for fucosylated as well as afucosylated glycan species (see data for Fc-gammaRIIIa binding).

### Example 7:

### Assessment of ADCC mediated by different glycan species of IgG1 antibodies (trastuzumab) - affinity chromatography

The ADCC mediated by the antibody populations generated in Example 3 was analyzed in a NK cell-line based *in vitro* ADCC assay, which was carried out as described in Example 2.

Results are shown in Fig. 6. Indicated is the relative ADCC when compared to the untreated trastuzumab sample. The data demonstrate that while agalactosylated trastuzumab species decrease ADCC mediated by the antibodies, bi-galactosylation of IgG1 (observed for sialylated glycan species as well as asialylated glycan species) improves ADCC mediation.

### Example 8:

### Assessment of Fc-gammaR binding mediated by hypergalactosylated species of different IgG1 antibodies

The binding of the monoclonal antibody samples to human Fc-gammaRIIIa was assessed in a competitive binding assay that uses a bead-based non-radioactive luminescent homogeneous proximity assay format (AlphaScreen®). In this assay, human glutathione S-transferase (GST)-tagged Fc-gamma receptor and antibody reference standard, control, and sample(s) are added to a 96-well plate, followed by the addition of biotinylated antibody streptavidin donor beads, and glutathione acceptor beads. In the absence of a competing antibody sample, donor bead-bound antibody and acceptor bead-bound Fc-gamma receptor interact to produce a luminescent signal. The sample disrupts the donor bead-bound antibody/Fcγ receptor interaction reducing the luminescent signal. The changes in luminescence are proportional to the amount of competing antibody. The results, expressed in luminescence counts, are plotted against antibody concentrations, and a four-parameter curve-fitting program is used to determine the activity of the sample(s) relative to the appropriate reference standard.

In order to confirm that the effects of hypergalactosylation on ADCC resulted from the increased interactions between the modified mAb Fc and Fc-gammaRIIIa expressed on the NK cells, a subset of rituximab (mAb2) and pertuzumab (mAb3) batches were analyzed in Fc-gammaRIIIa binding experiments. The results (Figure 7) demonstrate that hypergalactosylation of the antibodies has a pronounced increase in Fc-gammaRIIIa binding. The Fc-gammaRIIIa binding activities for the control and the hypergalactosylated mAb samples increased from 62 % to 103 % for rituximab (mAb2), from 73 % to 138 % for pertuzumab (mAb3), from 77 % to 160 % for mAb4 and from 100 % to 165 % for afucosylated mAb4 (mAb4 afu), respectively. Compared to the impact on Fc-gammaRIIIa binding, the effect of hypergalactosylation on ADCC activity was less pronounced. As shown in figure 7, the ADCC of the corresponding batches was increased from 55 % to 85 % for rituximab (mAb2), from 64 to 75 % for pertuzumab (mAb3) and from 90 % to 98 % for mAb4 for the control and hypergalactosylated mAb samples, respectively. The ADCC of the afucosylated variant of mAb4 (mAb4 afu) remained unchanged (100 % and 101 % for control and hypergalactosylated material, respectively).

## Claims

1. A population of galactoengineered recombinant antibodies of IgG1 isotype, comprising a relative frequency of at least 70 % of Fc bi-galactosylated antibodies.

2. The population of antibodies according to claim 1 comprising a relative frequency of less than 20 % of Fc afucosylated antibodies.

3. The population of antibodies according to claim 1 comprising a relative frequency of 80 - 95 % of Fc bi-galactosylated antibodies.

4. The population of antibodies according to claim 1 comprising a relative frequency of 7 - 16 % of Fc afucosylated antibodies.

5. The population of antibodies according to claim 1, wherein the antibody is selected from the group consisting of trastuzumab, rituximab, pertuzumab and obinutuzumab.

6. The population of antibodies according to claim 1, wherein the antibody is an *in vitro* galactoengineered antibody.

7. The population of antibodies according to claim 1, wherein the antibody is an *in vivo* galactoengineered antibody.

8. A population of galactoengineered recombinant antibodies of IgG1 isotype, comprising a relative frequency of at least 80 % of Fc galactosylated antibodies.

9. The population of antibodies according to claim 8, wherein the antibody is an *in vitro* galactoengineered antibody.

10. The population of antibodies according to claim 8, wherein the antibody is an *in vivo* galactoengineered antibody.

11. A method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) genetically engineering the host cell to improve protein galactosylation such that a population of said galactoengineered recombinant antibody is obtained, which comprises a relative frequency of at least 70 % of Fc bi-galactosylated antibodies.

12. A method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) genetically engineering the host cell to improve protein galactosylation such that a population of said galactoengineered recombinant antibody is obtained, which comprises a relative frequency of at least 80 % of Fc galactosylated antibodies.

13. A method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) isolating said population of the recombinant antibody,
c) enzymatically treating said population of recombinant antibodies with galactosyltransferase to obtain a population of galactoengineered recombinant antibodies, which comprises a relative frequency of at least 70 % of Fc bi-galactosylated antibodies and subsequent separation of the population of said galactoengineered recombinant antibodiesfrom said enzyme.

14. A method for the production of a population of galactoengineered recombinant antibodies, comprising the steps of
a) recombinantly producing an antibody of IgG1 isotype in a host cell, which comprises nucleic acid molecules encoding the antibody, to obtain a population of a recombinant antibody,
b) isolating said population of the recombinant antibody,
c) enzymatically treating said population of recombinant antibodies with galactosyltransferase to obtain a population of galactoengineered recombinant antibodies, which comprises a relative frequency of at least 80 % of Fc galactosylated antibodies and subsequent separation of the population of said galactoengineered recombinant antibodiesfrom said enzyme.

15. The method according to any one of claims 11 to 14, wherein the population of said galactoengineered recombinant antibodies comprises a relative frequency of less than 20 % of Fc afucosylated antibodies.

16. The method according to any one of claims 11 to 14, wherein the population of said galactoengineered recombinant antibodies comprises a relative frequency of 80 % to 90 % of Fc bi-galactosylated antibodies and a relative frequency of 7 - 16 % of Fc afucosylated antibodies.

17. The method according to any one of claims 11 to 14, wherein the host cell is a eukaryotic cell.

18. The method according to claim 17, wherein the eukaryotic cell is a CHO cell.

19. The method according to any one of claims 11 to 14, wherein the host cell is not genetically engineered to improve or impair protein fucosylation and the method according to the invention does not include the *in vitro* addition or *in vitro* cleavage of a fucose residue from the from the N-linked glycan structure of the antibodies.

20. The method according to any one of claims 11 to 14, wherein the antibodyis selected from the group consisting of trastuzumab, rituximab, pertuzumab and obinutuzumab.

21. Use of a method according to any one of claims 11 to 14 for improving ADCC mediated by said population of recombinant antibodies of IgG1 isotype.

22. Use of a method according to any one of claims 11 to 14 for improving the binding affinity of recombinant antibodies to Fc-gammaRIIa and Fc-gammaRIIIa.

23. The population of galactoengineered recombinant antibodies of IgG1 isotype according to any one of claims 1 to 8 or obtained by a method according to any one of claims 11 to 14 for mediation of ADCC.
